# EUROPEAN PATENT APPLICATION

(11) **EP 4 834 242 A1**
(43) Date of publication of application: **04.11.2026**
(21) Application number: 24910924.0
(22) Date of filing: 19.12.2024
(51) Int. Cl.: A61B 17/04, A61B 17/062

(54) **SUTURE APPARATUS**

(30) Priority: 28.12.2023 CN 202311844438
(71) Applicant: Shenzhen Bihe Medical Co., Ltd., Shenzhen, Guangdong 518063 (CN)
(72) Inventor: TANG, Lin, Shenzhen, Guangdong 518063 (CN)
(74) Representative: Michalski Hüttermann & Partner mbB
(86) International application number: PCT/CN2024/140611
(87) International publication number: WO 2025/139995

(57) **Abstract**

Disclosed is a suture apparatus. The suture apparatus further includes an actuating assembly, a needle hub, a first support member, and a second support member. The first support member is movably connected to the needle hub, the needle hub is connected to a suture needle, and the actuating assembly and the needle hub are detachably connected in a direction perpendicular to an axial direction. When the suture needle extends out, a distal end of the suture needle can be connected to a suture arm. After the actuating assembly is connected to the needle hub, the first support member is located outside the needle hub, and the second support member is located outside the actuating assembly, a straight line in an axial direction of the first support member and a straight line in an axial direction of the second support member are oppositely arranged in the direction perpendicular to the axial direction, and the first support member and the second support member are used for supporting the actuating assembly and the needle hub in the direction perpendicular to the axial direction. The actuating assembly and the needle hub that are connected to each other can axially move between the first support member and the second support member. When the first support member moves toward a proximal end, the first support member drives the needle hub to move towards the proximal end, and the needle hub drives the actuating assembly to move towards the proximal end until the actuating assembly is detached from the needle hub.

## Description

### Technical Field

The present invention relates to the technical field of medical devices, and particularly, to a suture apparatus.

### Background Art

Patent foramen ovale (PFO) was previously considered a benign and harmless condition. However, recent clinical studies have led to a consensus among experts that PFO is one of the causes of unexplained stroke, migraine, paradoxical embolism, and other conditions. Therefore, PFO occlusion or closure is required.

In the prior art, an objective of treatment is achieved by placing an occluder to occlude the foramen ovale. The existing occluder typically includes an integrated occlusion body composed of two occlusion discs. The two occlusion discs are respectively located on two sides of the membrane wall to fix the occluder relative to the membrane wall and achieve the function of occlusion. The two occlusion discs are in a frictional contact with the membrane wall, and it is difficult to adjust the position of the occluder relative to the membrane wall, thereby reducing the occlusion effect of the occluder on a defect of the membrane wall. In addition, the distance between the two occlusion discs of the integrated occlusion body is constant and cannot be adjusted. If the membrane wall is thin, the occluder may provide inadequate occlusion. If the membrane wall is thick, the occluder may excessively compress the membrane wall to cause damage, thereby increasing the risk of complications.

### Summary of the Invention

To overcome the foregoing defects in the prior art, the present invention provides an interventional suture apparatus.

The present invention solves the technical problems through the following technical solutions:
Embodiments of the present invention provide a suture apparatus, including a suture needle, a suture arm, and a suture thread. At least one end of the suture thread is located on the suture arm, a distal end of the suture needle is capable of being connected to the suture arm to extract the at least one end of the suture thread from the suture arm. The suture apparatus further includes an actuating assembly, a needle hub, a first support member, and a second support member. The first support member is movably connected to the needle hub.

The needle hub is connected to the suture needle. The actuating assembly and the needle hub are detachably connected in a direction perpendicular to an axial direction, and the actuating assembly controls the extension and retraction of the suture needle through the needle hub. When the suture needle extends out, the distal end of the suture needle is capable of being connected to the suture arm.

After the actuating assembly is connected to the needle hub, the first support member is located outside the needle hub, the second support member is located outside the actuating assembly, a straight line in an axial direction of the first support member and a straight line in an axial direction of the second support member are oppositely arranged in the direction perpendicular to the axial direction, and the first support member and the second support member are used for supporting the actuating assembly and the needle hub in the direction perpendicular to the axial direction. The actuating assembly and the needle hub that are connected to each other are capable of axially moving between the first support member and the second support member.

When moving toward a proximal end, the first support member drives the needle hub to move towards the proximal end, and the needle hub drives the actuating assembly to move towards the proximal end until the actuating assembly is detached from the needle hub.

In the foregoing suture apparatus, the first support member and the second support member are arranged. After the actuating assembly is connected to the needle hub, the first support member is located outside the needle hub, the second support member is located outside the actuating assembly, the straight line in the axial direction of the first support member and the straight line of the axial direction of the second support member are oppositely arranged in the direction perpendicular to the axial direction, and the first support member and the second support member are used for supporting the actuating assembly and the needle hub in the direction perpendicular to the axial direction. To be specific, the first support member is used for supporting the needle hub, the second support member is used for supporting the actuating assembly, and the first support member, the needle hub, the actuating assembly, and the second support member support each other on the same plane perpendicular to the axial direction, thereby preventing the four components from being detached from each other in the direction perpendicular to the axial direction without affecting the axial movement of the actuating assembly and the needle hub that are connected to each other between the first support member and the second support member. Consequently, this does not affect the axially moving actuation assembly in controlling the extension and retraction of the suture needle. Secondly, the first support member is movably connected to the needle hub, and the actuating assembly and the needle hub are detachably connected in the direction perpendicular to the axial direction. When moving towards the proximal end, the first support member drives the needle hub to move towards the proximal end. In this case, the first support member, the needle hub, the actuating assembly, and the second support member still support each other on the same plane perpendicular to the axial direction. Even if axial displacement of the four components occurs, the four components are still not detached from each other in the direction perpendicular to the axial direction. In addition, the needle hub can drive the actuating assembly to move towards the proximal end. Because the actuating assembly controls the extension and retraction of the suture needle through the needle hub, before the actuating assembly is detached from the needle hub, the actuating assembly can be driven first to move towards the proximal end, thereby reducing interference from the actuating assembly with other components of the suture apparatus and enhancing operational performance. Thirdly, by connecting the needle hub to the suture needle, the actuating assembly can be detached from the needle hub, so that the suture needle with the suture thread already pulled out (namely, the suture needle with the suture thread that has been already used for suturing in vivo) can be extracted from the body. In addition, by extracting the suture needle from the body, a portion connected to the suture needle of the suture thread can be withdrawn from the body. This makes it convenient for an operator to deliver a knotting device into the body along the portion of the suture thread that has been withdrawn from the body, or to directly knot the portion of the suture thread that has been withdrawn from the body and push it into the body to complete the suturing and fixation.

### Brief Description of the Drawings

The present invention will be further described in detail below with reference to the drawings and embodiments.
Fig. 1 is a schematic structural diagram of a suture apparatus or a delivery apparatus according to an embodiment;
Fig. 2 is a schematic diagram of a partial structure of a suture apparatus or delivery apparatus including a suture arm in an expanded state according to an embodiment;
Fig. 3 is a schematic diagram of an exploded structure of a suture apparatus or a delivery apparatus according to an embodiment;
Fig. 4 is a schematic diagram of an enlarged structure of the portion A in Fig. 3;
Fig. 5 is a schematic diagram of a partial structure of a suture apparatus or delivery apparatus according to an embodiment;
Fig. 6 is a schematic sectional diagram of a partial structure of a suture apparatus or delivery apparatus including a first control member in an initial state according to an embodiment;
Fig. 7 is a schematic sectional diagram of a partial structure of a suture apparatus or delivery apparatus including a first control member in a locked state according to an embodiment;
Fig. 8a is a schematic structural diagram of a sliding block assembly 8 according to an embodiment; and Fig. 8b is a schematic diagram of an exploded structure of the sliding block assembly 8 according to an embodiment of the present invention;
Fig. 9 is a schematic diagram of a simple structure showing the movement relationship between a first control member and a first connecting member according to an embodiment;
Fig. 10 is a schematic diagram of a simple structure showing the movement relationship between a first control member and a first connecting member according to another embodiment;
Fig. 11 is a schematic diagram of a partial structure of a suture apparatus or delivery apparatus according to an embodiment;
Fig. 12a is a schematic sectional diagram of a partial structure of a suture apparatus or delivery apparatus including a first control member in an initial state according to an embodiment; and Fig. 12b is a schematic sectional diagram of a partial structure of the suture apparatus or delivery apparatus including the first control member in a locked state according to an embodiment;
Fig. 13 is a schematic diagram of a partial structure of a suture apparatus or delivery apparatus including a suture arm in an expanded state according to another embodiment;
Fig. 14 is a schematic diagram of a partial structure of a suture apparatus or delivery apparatus according to an embodiment;
Fig. 15a is a schematic sectional diagram of a partial structure of a suture apparatus or delivery apparatus at a certain moment according to an embodiment; Fig. 15b is a schematic sectional diagram of a partial structure of the suture apparatus or delivery apparatus at another moment according to an embodiment; and Fig. 15c is a schematic sectional diagram of a partial structure of the suture apparatus or delivery apparatus at still another moment according to an embodiment;
Fig. 16a is a schematic diagram of a partial structure of a suture apparatus or delivery apparatus from a perspective according to an embodiment; Fig. 16b is a schematic diagram of a partial structure of the suture apparatus or delivery apparatus from another perspective according to an embodiment; and Fig. 16c is a schematic sectional diagram of a partial structure of the suture apparatus or delivery apparatus from still another perspective according to an embodiment;
Fig. 17a is a schematic sectional diagram of a partial structure of a suture apparatus or delivery apparatus according to an embodiment; and Fig. 17b is a schematic diagram of a partial structure in Fig. 17a;
Fig. 18a is a schematic sectional diagram of a partial structure of a suture apparatus or delivery apparatus according to an embodiment; Fig. 18b is a schematic structural diagram of a thread cutter in Fig. 18a from a perspective; and Fig. 18c is a schematic structural diagram of the thread cutter in Fig. 18a from another perspective;
Fig. 19a is a schematic structural diagram of matching between a first control member in an initial state and a thread spool according to an embodiment; Fig. 19b is a schematic structural diagram of matching between the first control member in a locked state and the thread spool according to an embodiment; and Fig. 19c is a schematic diagram of connection between the thread spool and a suture thread according to an embodiment;
Fig. 20 is a schematic diagram of an enlarged structure of the portion B in Fig. 3;
Fig. 21 is a schematic diagram of a partial exploded structure of a suture apparatus or delivery apparatus according to an embodiment;
Fig. 22a is a schematic structural diagram of a first control member in an initial state according to an embodiment; and Fig. 22b is a schematic structural diagram of the first control member in a locked state according to an embodiment;
Fig. 23a is a schematic sectional diagram of a partial structure of a suture apparatus or delivery apparatus including a first control member in an initial state according to an embodiment; Fig. 23b is a schematic sectional diagram of the first control member in the initial state in Fig. 23a; Fig. 23c is a schematic sectional diagram of a partial structure of the suture apparatus or delivery apparatus including the first control member in a locked state according to an embodiment; and Fig. 23d is a schematic sectional diagram of the first control member in the locked state in Fig. 23c;
Fig. 24 is a schematic sectional diagram of a partial structure of a suture apparatus or delivery apparatus according to an embodiment;
Fig. 25a is a schematic diagram of a partial structure of a suture apparatus or delivery apparatus according to an embodiment; and Fig. 25b is a schematic diagram of a knob in Fig. 25a;
Fig. 26a is a schematic diagram of a partial structure of a suture apparatus or delivery apparatus according to an embodiment; and Fig. 26b is a schematic diagram of a partial structure in Fig. 26a;
Fig. 27 is a schematic diagram of a partial exploded structure of a suture apparatus or delivery apparatus according to an embodiment;
Fig. 28 is a schematic structural diagram of a receiving structure according to an embodiment; and

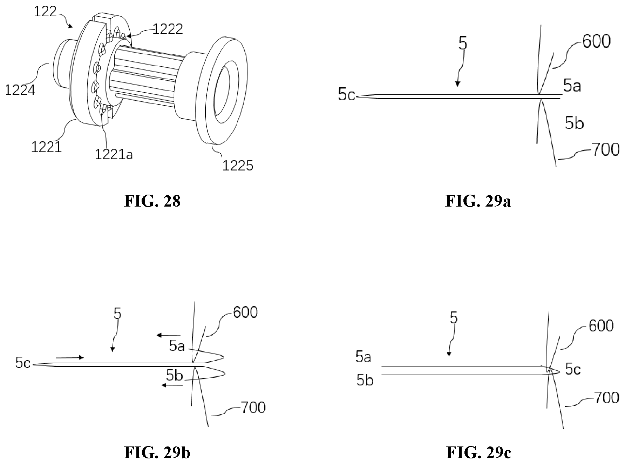

Fig. 29a is a schematic diagram of a state of a suture thread at a certain moment in a process of suturing a foramen ovale by a suture apparatus or a delivery apparatus according to an embodiment; Fig. 29b is a schematic diagram of a state of the suture thread at a certain moment in the process of suturing the foramen ovale by the suture apparatus or the delivery apparatus according to an embodiment; and Fig. 29c is a schematic diagram of a state of the suture thread at a certain moment in the process of suturing the foramen ovale by the suture apparatus or the delivery apparatus according to an embodiment.

### Detailed Description of the Invention

To more clearly understand the technical features, objectives, and effects of the present invention, specific embodiments of the present invention will be described in detail with reference to the accompanying drawings.

Exemplary embodiments of the present invention will be described in further detail below with reference to the accompanying drawings. Although the exemplary embodiments of the present invention are illustrated in the accompanying drawings, it should be understood that the present invention may be implemented in various ways and should not be limited to the embodiments described here. Rather, these embodiments are provided, so that the present invention will be understood more thoroughly, and the scope of the present invention is fully conveyed to those skilled in the art.

It should be understood that the terms used herein are only for the purpose of describing specific exemplary embodiments and are not intended to be limitation. Unless otherwise clearly specified in context, the singular forms "a", "an", and "the" used herein may also represent plural forms. The terms "include", "comprise", "contain", and "have" are inclusive and therefore indicate the presence of a stated feature, step, operation, element, and/or component, but do not exclude the presence or addition of one or more other features, steps, operations, elements, components, and/or combinations thereof. The methods, procedures, and operations described herein are not to be construed as requiring them to be performed in the specific order described or indicated, unless the order of execution is explicitly specified. It should also be understood that other or alternative steps may be used.

Although the terms "first", "second", "third", and the like may be used herein for describing multiple elements, components, regions, layers, and/or sections, these elements, components, regions, layers, and/or sections should not be limited by these terms. These terms may be only used for distinguishing one element, component, region, layer, or section from another region, layer, or section. Unless otherwise clearly specified in context, the terms, such as "first" and "second", and other numerical terms do not imply order or sequence when used herein. Therefore, a first element, component, region, layer, or section discussed below may be referred to as a second element, component, region, layer, or section without departing from the teachings of the exemplar embodiments.

For ease of description, spatial relation terms may be used herein for describing the relationship between one element or feature and another element or feature as shown in the figure. These relation terms include, for example, "inside", "outside", "inner side", "outer side", "under", "below", "on", and "above". These spatial relation terms are intended to include different orientations of an apparatus in use or operation, in addition to the orientations depicted in the figure. For example, if the apparatus in the figure is flipped, an element described as "under another element or feature" or "below another element or feature" is subsequently be oriented as "on another element or feature" or "above another element or feature". Therefore, the exemplar term "below" may include both above and below. The apparatus may be oriented in another way (rotated 90 degrees or in another direction) and is correspondingly interpreted by using the spatial relation descriptors.

It should be noted that the terms "distal end" and "proximal end" are used as locative terms, which are commonly used in the field of interventional medical devices. "Distal end" refers to an end that is away from an operator during operation, while "proximal end" refers to an end that is close to the operator during operation. "Axial direction" refers to a direction parallel to a connecting line between a distal end center and a proximal end center of a medical device; and "radial direction" refers to a direction perpendicular to the axial direction.

A suture apparatus in the embodiments of the present invention includes, but is not limited to, those used for suturing a patent foramen ovale, valve edge-to-edge suturing and repair, chordae tendineae repair, vascular suturing, and the like.

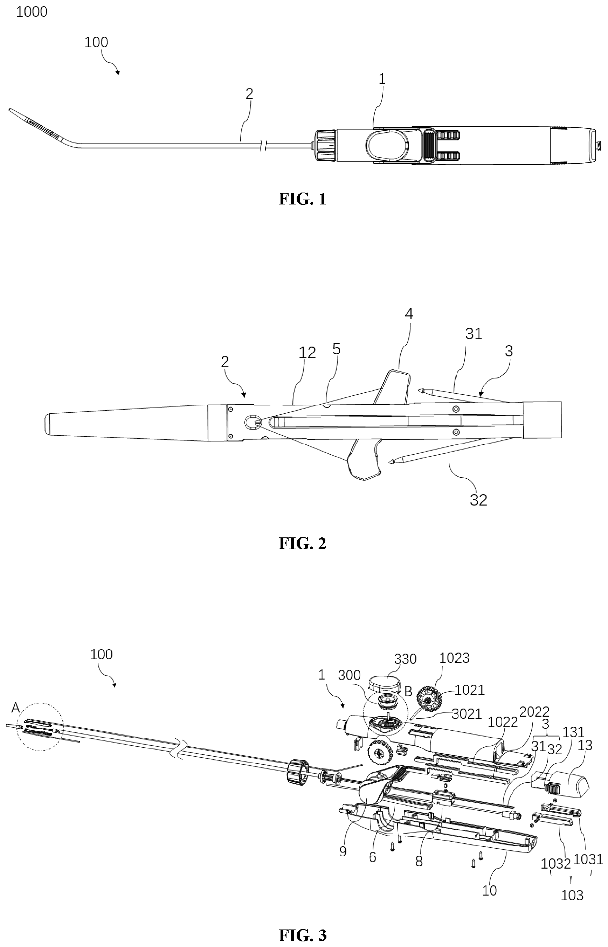

Refer to Fig. 1 and Fig. 2. This embodiment provides a suture apparatus 100, including a handle 1 and a sheath 2 that are connected to each other. The suture apparatus 100 further includes a suture needle 3, a suture arm 4, and a suture thread 5. At least one end of the suture thread 5 may be located on the suture arm 4. The suture arm 4 switches between a delivered state and an expanded state. When the suture arm is in the expanded state, a distal end of the suture needle 3 can be connected to the suture arm 4, to extract the at least one end of the suture thread 5 from the suture arm 4. The suture needle 3 and the suture thread 5 are arranged inside the sheath 2, the suture arm 4 is arranged at a distal end or a position close to the distal end of the sheath 2, and a proximal end of the suture needle 3 is arranged on the handle 1. Fig. 1 shows the suture arm 4 in the delivered state, that is, the suture arm 4 is not expanded and the suture needle 3 does not extend out. Fig. 2 shows the suture arm 4 in the expanded state, that is, the suture needle 3 has already extended out and the distal end of the suture needle 3 is about to be connected to the suture arm 4.

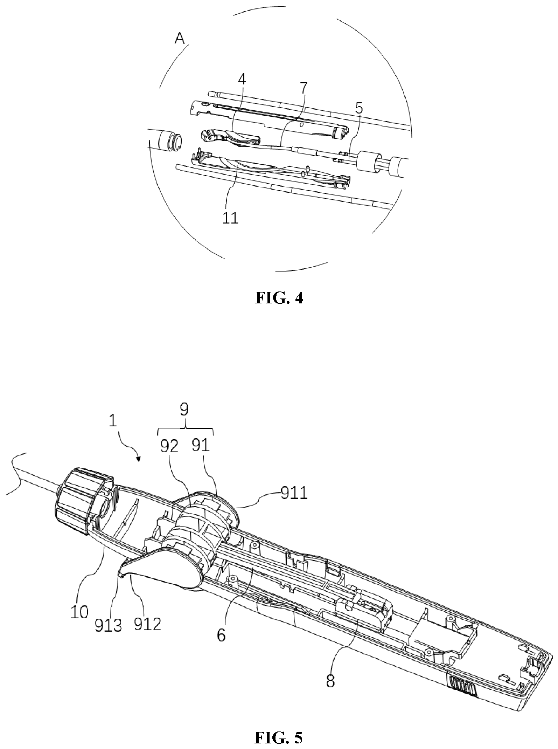

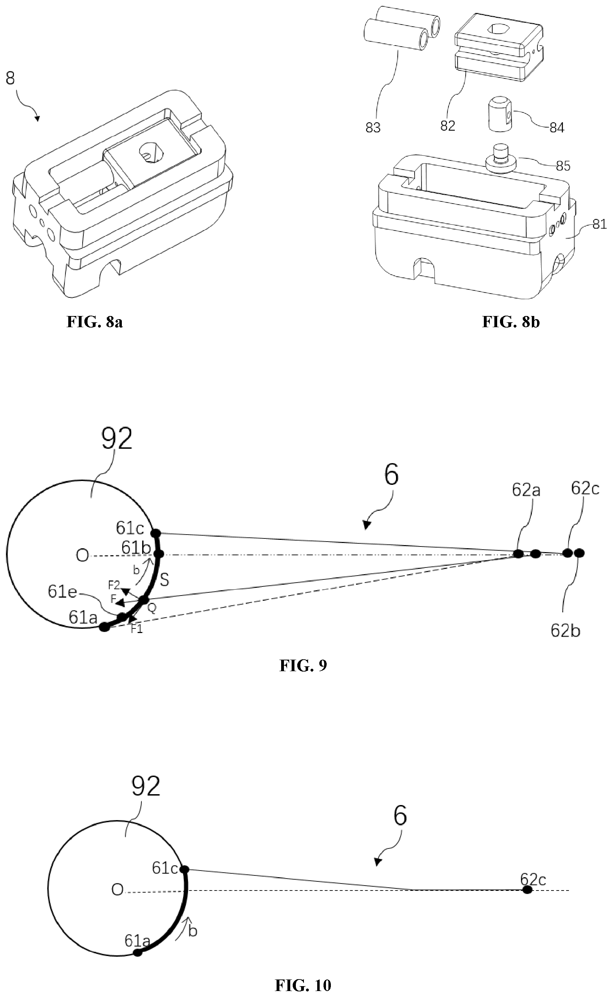

Refer to Fig. 3 to Fig. 7. In one embodiment, the suture apparatus 100 further includes a first connecting member 6, a second connecting member 7, a sliding block assembly 8, and a first control member 9. As shown in Fig. 8a and Fig. 8b, the sliding block assembly 8 includes a first sliding block 81, a second sliding block 82, and an elastic member 83 that are connected to each other. The second sliding block 82 and the elastic member 83 are axially arranged inside the first sliding block 81, and the second sliding block 82 can move relative to the first sliding block 81. As shown in Fig. 4, Fig. 6, and Fig. 7, two ends of the first connecting member 6 are respectively connected to the first sliding block 81 and the first control member 9, two ends of the second connecting member 7 are respectively connected to the suture arm 4 and the second sliding block 82 to control movement of the first sliding block 81 through the first control member 9, and the first sliding block 81 drives the second sliding block 82 to move through the elastic member 83 to control movement of the suture arm 4, that is, to switch from the delivered state to the expanded state.

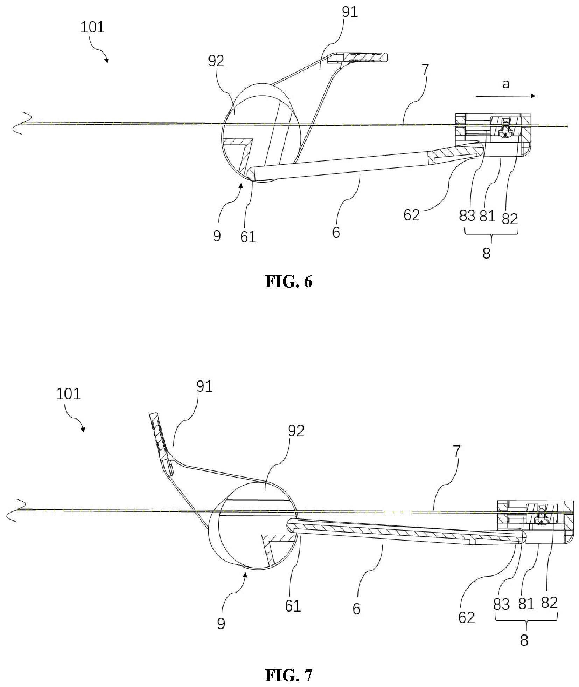

The first control member 9 has an initial state and a locked state. Fig. 6 shows the first control member 9 in the initial state, and Fig. 7 shows the first control member 9 in the locked state. During movement of the first control member 9 from the initial state to the locked state, the suture arm 4 switches from the delivered state to the expanded state, the first control member 9 drives the first sliding block 81 through the first connecting member 6 to axially move in a first direction a, to form a first movement stroke relative to a handle housing, the first sliding block 81 drives the second sliding block 82 through the elastic member 83 to axially move in the first direction a, to form a second movement stroke relative to the handle housing, and the first movement stroke is greater than the second movement stroke, to allow elastic deformation of the elastic member 83 inside the first sliding block 81.

Referring to Fig. 2 and Fig. 4, in this embodiment, the suture arm 4 switches from the delivered state to the expanded state in the following manner: the second connecting member 7 is enabled to move in the first direction a, to drive the suture arm 4 to move in the first direction a along a sliding track 11 on an inner wall of the sheath 2, and the sheath 2 is further provided with an open hole 12, so that the suture arm 4 can extend through the open hole 12 during movement and is slowly expanded until the suture arm 4 moves to abut against an inner wall of the open hole 12 in the sheath 2. In this embodiment, the first direction a is a direction from a distal end to a proximal end. In another embodiment, the first direction a may alternatively be a direction from the proximal end to the distal end.

It may be understood that, because the second sliding block 82 is connected to the suture arm 4 through the second connecting member 7, the second sliding block 82 axially moves in the first direction a, to drive the second connecting member 7 and the suturing arm 4 to axially move in the first direction a until the suture arm 4 moves to abut against the inner wall of the open hole 12 in the sheath 2. In this case, the second connecting member 7 and the suture arm 4 can no longer move in the first direction a, the suture arm 4 is in the expanded state, and it may be considered that the first control member 9 is in a pre-locked state at this moment. The pre-locked state is a certain state between the initial state and the locked state. In the pre-locked state, the second sliding block 82 connected to the second connecting member 7 can no longer move in the first direction a due to the second connecting member 7 and is stationary relative to a housing, that is, the second sliding block 82 has completed the second movement stroke. However, in this case, the suture arm 4 can still restore to the delivered state under the action of external force. Therefore, the suture arm 4 is not in a truly locked state, and the first control member 9 has only reached the pre-locked state and has not reached the locked state. Next, if the first control member 9 is further moved in the original direction, the first connecting member 6 is enabled to drive the first sliding block 81 to continue to axially move in the first direction a, so as to achieve the first movement stroke greater than the second movement stroke, which allows the elastic deformation of the elastic member 83 between the first sliding block 81 and the second sliding block 82 inside the first sliding block 81. When the first control member 9 reaches the locked state, because the balance of a force applied by the first control member 9 in the locked state to the first sliding block 81, a force applied by the elastic member undergoing the foregoing elastic deformation to the first sliding block 81, and the friction applied by a handle housing 10 to the first sliding block 81 can be achieved, the first control member 9, the first connecting member 6, the first sliding block 81, the elastic member 83, and the second sliding block 82 are all stationary relative to the handle housing 10, that is, the first control member 9, the first connecting member 6, the first sliding block 81, the elastic member 83, and the second sliding block 82 can remain fixed relative to each other, which can prevent the suture arm 4 from restoring to the delivered state or an uncertain state between the delivered state and the expanded state. In this way, the suture arm 4 is fixed relative to the handle housing 10. That is, the suture arm 4 in the expanded state is locked by locking the first control member 9, to avoid uncertain movement of the second sliding block 82 under the action of unexpected external force, which facilitate connection between the suture needle 3 and the suture arm 4 and increase the success rate of operation.

It may be understood that before undergoing the foregoing elastic deformation, the elastic member 83 may have a small initial compressive elastic deformation. The elastic member 83 having the initial elastic deformation is placed inside the first sliding block 81, and two ends of the elastic member are respectively connected to the first sliding block 81 and the second sliding block 82. Because the initial elastic deformation is too small to overcome the static friction between the first sliding block 81 and the second sliding block 82 and to drive the suture arm 4 to move, the elastic member cannot drive the second sliding block 82 and the suture arm 4 to move before undergoing the foregoing elastic deformation, so that the suture arm 4 remains in the delivered state. In another embodiment, before undergoing the foregoing elastic deformation, the elastic member 83 may alternatively be in a natural state and has no initial elastic deformation. In this case, the elastic member may be located on a proximal side of the second sliding block or may be located on a distal side of the second sliding block. In another embodiment, before undergoing the foregoing elastic deformation, the elastic member 83 may have a small initial tensile elastic deformation. In this case, the second sliding block may be located on a distal side of the elastic member, a proximal end of the elastic member is connected to the first sliding block, and a distal end of the elastic member is connected to the second sliding block.

Referring to Fig. 5, in this embodiment, the first control member 9 includes a first operating portion 91 and a main body portion 92 that are connected to each other. The first operating portion 91 is operated by an operator, rotating the first operating portion 91 can drive the main body portion 92 to rotate, and the first connecting member 6 is connected to the main body portion 92. In another embodiment, the first operating portion 91 may be omitted, and the main body portion 92 is directly rotated. When locking is required, the main body portion 92 may be locked through an additional locking member or directly locked by hand.

Referring to Fig. 9, which is a schematic diagram of a trajectory of the first control member 9 moving from the initial state to the locked state, and in the figure, 61 denotes a connecting point between the first connecting member 6 and the main body portion 92, and 62 denotes a connecting point between the first connecting member 6 and the first sliding block 81. As the main body portion 92 rotates, the connecting point 61 between the first connecting member 6 and the main body portion 92 rotates in a direction b from 61a to 61b and finally to 61c. The connecting point 61 undergoes arc movement on the main body portion 92, to form an arc path S. The connecting point 62 between the first connecting member 6 and the first sliding block 81 axially moves from 62a to 62b and finally to 62c. Through such a design, arc movement is converted into linear movement, thereby improving operability and user experience.

Specifically, the arc path S includes the starting end 61a and the ending end 61c. The arc path S further include the transition point 61b, and the transition point 61b is located between the starting end 61a and the ending end 61c. When an end that is connected to the main body portion 92 of the first control member 9 of the first connecting member 6 is located at the transition point 61b, the axial distance between a central rotation point O of the main body portion 92 of the first control member 9 and the first sliding block 81 is at its maximum. In this case, the first sliding block 81 moves the maximum axial distance, and the elastic member 83 undergoes the greatest elastic deformation. However, the first control member 9 has not reached a locked position at this moment, that is, has not reached the locked state. The first control member 9 continues to move in the original direction, to drive the first sliding block 81 to move in a direction opposite to the first direction a. In this case, the elastic deformation of the elastic member 83 decreases slightly until the first control member 9 reaches the locked position. At the locked position, the first control member 9 is limited or locked by another element (such as the handle housing), so that the elastic member 83 undergoing the elastic deformation cannot drive the first sliding block 81 to move in the direction opposite to the first direction a. In this way, the first control member 9 remains in the locked state. It may be understood that in the foregoing process in which the elastic member 83 undergoing the elastic deformation applies a force to the first sliding block 81, to drive the first sliding block 81 to move in the direction opposite to the first direction a, even if the first sliding block 81 moves a certain distance in the direction opposite to the first direction a, as long as the first movement stroke is greater than the second movement stroke, that is, the distance that the first sliding block 81 moves in the first direction a is still longer than the distance that the second sliding block 82 moves in the first direction a, in this case, the elastic member 83 undergoing the elastic deformation still applies a force to the second sliding block 82 in the first direction a, so that the second sliding block 82 remains in a stationary state, and the suture arm 4 connected to the second sliding block 82 remains in the expanded state.

In Fig. 9, in the pre-locked state, the connecting point between the first connecting member 6 and the main body portion 92 is located at a pre-locked point 61e, and the pre-locked point 61e may be located at a position that is on the arc path S and that is between the starting end 61a and the transition point 61b. During movement of the connecting point between the first connecting member 6 and the main body portion 92 from the starting end 61a to the pre-locked point 61e, the first sliding block 81 drives the second sliding block 82 to move together until the second blocking block 82 completes the second movement stroke, so that the suture arm 4 can be driven to move from the delivered state to the expanded state. However, the suture arm 4 cannot be locked in the expanded state yet. Because no special locking member rather than the handle housing 10 is designed, the suture arm can still move to the delivered state under the action of unexpected external force. During movement of the connecting point between the first connecting member 6 and the main body portion 92 from the pre-locked point 61e to the ending end 61c, the elastic member 83 undergoes the elastic deformation inside the first sliding block 81 until the first control member 9 is in the locked state. In this case, the suture arm 4 is also in the locked state and cannot move relative to the handle housing 10. Therefore, only when the connecting point between the first connecting member 6 and the main body portion 92 is located at a position after the pre-locked point 61e, the first control member 9 can be in the locked state. It may be understood that the transition point 61b is located between the pre-locked point 61e and the ending end 61c.

Referring to Fig. 3 and Fig. 5, the handle 1 includes a handle housing 10, the sliding assembly 8, the first connecting member 6, and the main body portion 92 are arranged inside the handle housing 10, and the first operating portion 91 extends through the handle housing 10 and is arranged outside the handle housing 10. As shown in Fig. 5, when the first operating portion 91 is in a locked state, the first operating portion 91 abuts against the handle housing 10. Refer now to Fig. 5, Fig. 11, and Fig. 19a. Specifically, the first operating portion 91 includes a first arm 911, a second arm 912, and a connecting arm 913. The connecting arm 913 is connected to one end of the first arm 911 and one end of the second arm 912, and the other end of the first arm 911 and the other end of the second arm 912 are separately connected to the main body portion 92. The operator can hold the connecting arm 913 by hand to operate movement of the first operating portion 91. The connecting arm 913 is used for abutting against the handle housing 10.

As shown in Fig. 7 and Fig. 9, the first control member 9 rotates continuously in a direction b until the first control member 9 abuts against the handle housing 10, and the handle housing 10 prevents the first control member 9 from continuing to rotate in the direction b. The force generated through the elastic deformation of the elastic member 83 cannot cause the first control member 9 to rotate in a direction opposite to the direction b either, so that the balance of forces is achieved. In this way, the first control member 9 is locked. In another embodiment, a method for locking the first control member 9 may alternatively be implemented by arranging an additional locking member and connecting the locking member to the first control member 9.

Refer to Fig. 9 again. In this embodiment, because no additional locking member rather than the handle housing is designed for locking the first control member 9, the locking of the first control member 9 is achieved through the handle housing 10 abutting against the first control member 9. In addition, when the first control member 9 is operated, the first control member 9 needs to be rotated to move from the initial state to the locked state, and in this process, one end connected to the first control member 9 of the first connecting member 6 undergoes arc movement on the first control member 9. Therefore, in this embodiment, when the first control member 9 is in the locked state, the end connected to the main body portion 92 of the first control member 9 of the first connecting member 6 needs to be located at the transition point 61b or at a certain position (such as 61c) after the transition point 61b. In this way, locking performance can be ensured. If the end that is connected to the main body portion 92 of the first control member 9 of the first connecting member 6 is located a certain point Q before the transition point 61b and no external force is applied to the first control member 9, then in a force analysis diagram, at the point Q, the elastic member 83 undergoing the elastic deformation applies a force F to the main body portion 92 of the first control member 9 through the first connecting member 6, the force F has a component force F1 in a circumferential direction and in the direction opposite to the direction b, and the component force F1 can drive the end that is connected to the main body portion 92 of the first control member 9 of the first connecting member 6 to move in the direction opposite to the direction b, which easily causes the end connected to the main body portion 92 of the first control member 9 of the first connecting member 6 to return to the starting end 61a. Therefore, the balance of forces cannot be achieved at the point Q, and the first control member 9 cannot reach the locked state.

The main body portion 92 has a central rotation point O, and the main body portion 92 rotates around the central rotation point O. When the end that is connected to the main body portion 92 of the first control member 9 of the connecting member 6 is located at the transition point 61b, the transition point 61b between the first connecting member 6 and the main body portion 92, the connecting point 62b between the first connecting member 6 and the first sliding block 81, and the central rotation point O are on a same straight line.

In another embodiment, when the first control member 9 reaches the locked position, the end that is connected to the main body portion 92 of the first control member 9 of the first connecting member 6 is also just located at the transition point 61b, that is, the ending end 61c coincides with the transition point 61b.

In Fig. 9, the first connecting member 6 is generally in the shape of a straight line. Referring to Fig. 10, in an embodiment, the first connecting member 6 is generally in the shape of a broken line. In another embodiment, the first connecting member 6 may also be generally in a shape of a curve.

In one embodiment, two ends of the first connecting member 6 are respectively pivotally connected to the first control member 9 and the first sliding block 81 so as to achieve a more flexible connection, which facilitates connecting the first control member 6 to the first control 9 and the first sliding block 81 at different angles. This facilitates relative movement between the first control member 9, the first connecting member 6, and the first sliding block 81.

Referring to Fig. 7 and Fig. 8b again, in one embodiment, the second sliding block 82 is arranged inside the first sliding block 81, a fixing post 84 is arranged inside the second sliding block 82, a proximal end of the second connecting member 7 first extends through the first sliding block 81, then enters the second sliding block 82, passes through the fixing post 84, and exits the second sliding block 82. By rotating the fixing post 84, the second connecting member 7 is wound around the fixing post 84. Finally, the fixing post 84 is fixed through a screw 85, thereby fixing the second connecting member 7 to the second sliding block 82.

In one embodiment, the first direction a is a movement direction from a first end to a second end. Referring to Fig. 6 and Fig. 7, in this embodiment, the first direction a is a direction from the distal end to the proximal end. The elastic member 83 is a compression-type elastic member, and the elastic member 83 is arranged on a side close to the distal end inside the first sliding block 81. The compression-type elastic member (such as a compression spring) may be understood as an elastic element that deforms by shortening when compressed. The elastic element tends to restore from the deformation, thereby storing energy generated through deformation. In another embodiment, the elastic member 83 is a tension-type elastic member, and the elastic member 83 is arranged on a side close to the proximal end inside the first sliding block 81. The tension-type elastic member (such as a tension spring) may be understood as an elastic element that deforms by elongating when stretched. The elastic element tends to restore from the deformation, thereby storing energy generated through deformation. It may be understood that the first direction a may alternatively be a direction from the proximal end to the distal end.

The suture apparatus 100 includes a first control assembly (or a suture arm controlling assembly) 101 and a second control assembly (or a needle hub controlling assembly) 102. Referring to Fig. 6 and Fig. 7, the first control assembly 101 includes the first connecting member 6, the second connecting member 7, the sliding assembly 8, and the first control member 9. The first control assembly 101 is used for controlling the suture arm 4 to switch between the delivered state and the expanded state. Referring to Fig. 2, Fig. 3, Fig. 11, and Fig. 12, the second control assembly 102 is connected to the suture needle 3 and is used for controlling the extension and retraction of the suture needle 3. When the suture needle 3 extends out, the distal end of the suture needle 3 can be connected to the suture arm 4.

The first control member 9 has a through hole 93 axially extending through the first control member. When the first control member 9 is in the locked state, the second control assembly 102 enters the through hole 93 and moves axially. When the first control member 9 is in the initial state, the second control assembly 102 is prevented from entering the through hole 93, thereby limiting the axial movement of the second control assembly 102. Through such an arrangement, only when the suture arm 4 is in the expanded state, the suture needle 3 can extend out to be connected to the suture arm 4. This can ensure that the suture arm 4 is expanded first and then the suture needle 3 extends out, and prevents the suture needle 3 from extending out when the suturing arm 4 is not expanded yet and prevents the suture needle 3 from causing damage to tissue.

In this embodiment, the first control member 9 is arranged on a distal side of the second control assembly 102. The second control assembly 102 drives the suture needle 3 to extend towards the distal end, so that the distal end of the suture needle 3 extends through the through hole 93 and enters a proximal end of the suture arm 4. Further, the at least one end of the suture thread 5 is extracted from the suture arm 4.

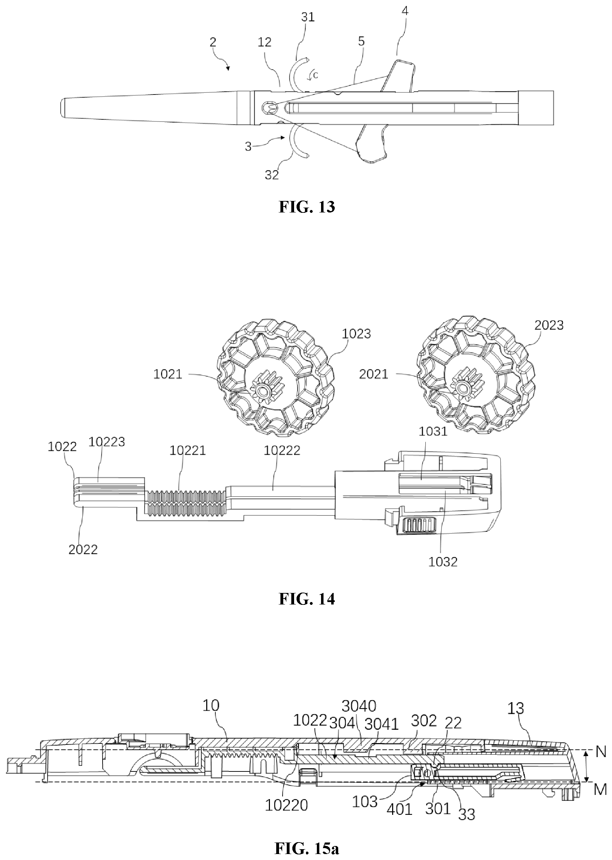

Referring to Fig. 13, in another embodiment, the distal end of the suture needle 3 in a delivered state can be restrained by the sheath 2. The second control assembly 102 drives the suture needle 3 to move towards the proximal end until the suture needle moves to the position of the open hole 12. A distal end portion of the suture needle 3 extends outwards in an everted shape. The second control assembly 102 continues to drive the suture needle 3 to move towards the proximal end until the suture needle 3 is connected to the suture arm 4. The second control assembly 102 further drives the suture needle 3 to move towards the proximal end, the distal end of the suture needle 3 is retracted in a direction C to be detached from the suture arm 4, so as to extract the at least one end of the suture thread 5 from the suture arm 4 until the distal end of the suture needle 3 enters the sheath 2 again. In addition, the everted distal end portion of the suture needle 3 is straightened. It may be understood that in this embodiment, the first control member 9 is arranged on a proximal side of the second control assembly 102. Only when the first control member 9 is in the locked state, a proximal end of the second control assembly 102 can enter the through hole 93 and axially move.

Referring to Fig. 3, Fig. 12a, Fig. 12b, and Fig. 14, the second control assembly 102 includes a first gear 1021, a first rack 1022, and a needle hub 103. The first gear 1021 and the first rack 1022 mesh with each other, and the needle hub 103 is connected to the suture needle 3 and the first rack 1022. The first gear 1021 is rotated to drive the needle hub 103 to axially move through the first rack 1022, thereby controlling the extension and retraction of the suture needle 3. When the suture needle 3 extends out, the distal end of the suture needle 3 is connected to the suture arm 4, thereby precisely controlling the movement of the suture needle 3 and improving the operability. Further, the second control assembly 102 further includes a second operating portion (or a gear operating portion) 1023. The second operating portion 1023 is connected to the first gear 1021, and the second operating portion 1023 is operated to drive the first gear 1021 to rotate.

In one embodiment, the first rack 1022 includes a meshing section 10221 and a connecting section 10222. The meshing section 10221 and the connecting section 10222 are connected in the axial direction. The meshing section 10221 is matched with the first gear 1021, and the connecting section 10222 is connected to the proximal end of the suture needle 3. The meshing section 10221 is a groove structure of the first gear 1022, and the first gear 1021 is restricted to move inside the groove structure.

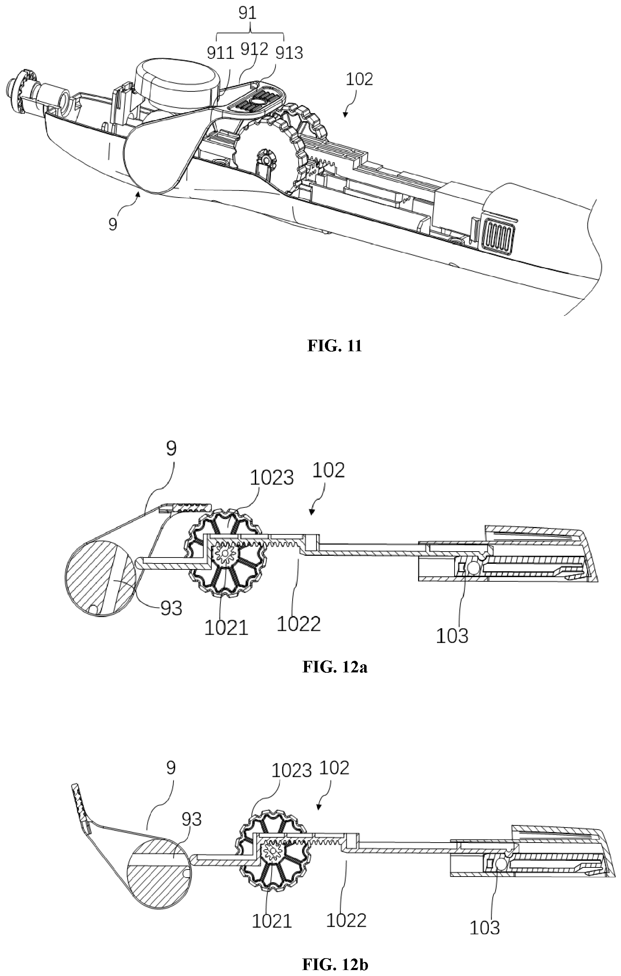

In one embodiment, the first gear 1022 includes an inserting section 10223 and a meshing section 10221. The inserting section 10223 and the meshing section 10221 are connected in the axial direction, the meshing section 10221 is matched with the first gear 1021, and the inserting section 10223 is used for entering the through hole 93 in the first control member 9 in Fig. 12.

In another embodiment, the first rack 1022 may include a meshing section 10221, a connecting section 10222, and an inserting section 10223 that are sequentially connected in the axial direction.

Referring to Fig. 2, Fig. 3, Fig. 13, and Fig. 14, in one embodiment, the suture needle 3 includes a first suture needle 31 and a second suture needle 32, and the needle hub 103 includes a first needle hub 1031 and a second needle hub 1032. The first suture needle 31 is connected to the first needle 1031, and the second suture needle 32 is connected to the second needle hub 1032. The second control assembly 102 further includes a second gear 2021 and a second rack 2022 that are matched with each other. The first gear 1021 is rotated to drive the first needle hub 1031 to axially move through the first rack 1022, thereby controlling extension and retraction of the first suture needle 31. The second gear 2021 is rotated to drive the second needle hub 1032 to axially move through the second rack 2022, thereby controlling extension and retraction of the second suture needle 32. In this way, the first suture needle 31 and the second suture needle 32 can be operated separately, so that the flexibility is high and the operability is improved.

Referring to Fig. 3 and Fig. 14, the first gear 1022 and the second gear 2022 are arranged side by side inside the handle housing 10, the first gear 1021 is connected to the second gear 2021 through a connecting rod 3021, the connecting rod 3021 extends through the first gear 1021 and the second gear 2021, two ends of the connecting rod 3021 are fixed onto the handle housing 10, and the first gear 1021 and the second gear 2021 separately rotate around the connecting rod 3021.

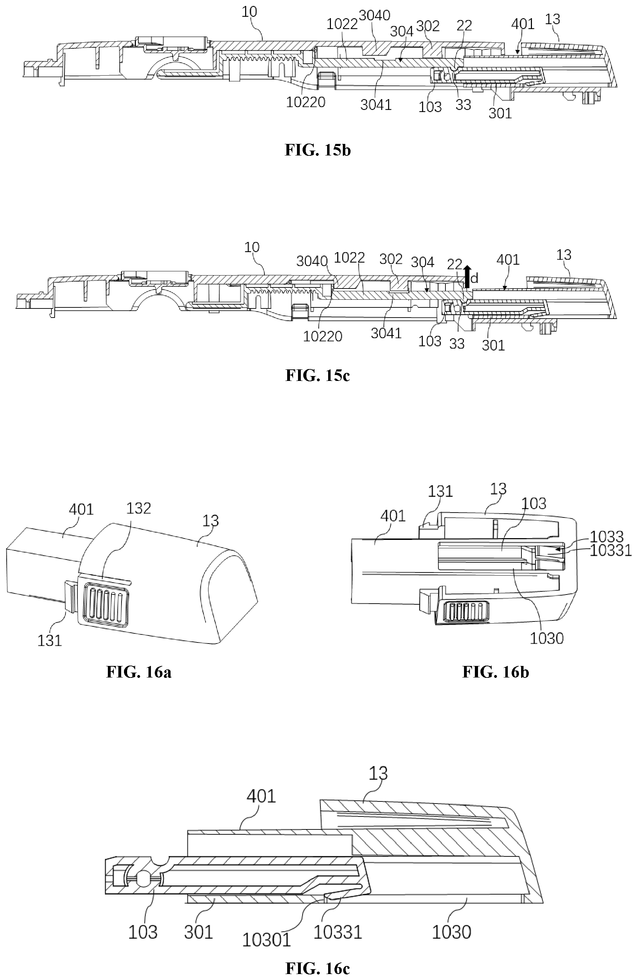

Referring to Fig. 15a, Fig. 15b, and Fig. 15c, in one embodiment, the suture apparatus 100 further includes an actuating assembly 304, a first support member 301, and a second support member 302. The first support member 301 is movably connected to the needle hub 103, and the actuating assembly 304 is used for controlling the extension and retraction of the suture needle 3 from the sheath. When the suture needle 3 extends out, the distal end of the suture needle 3 is connected to the suture arm 4, the needle hub 103 is connected to the suture needle 3, and the actuating assembly 304 and the needle hub 103 are detachably connected in a direction perpendicular to the axial direction.

After the actuating assembly 304 is connected to the needle hub 103, the first support member 301 is located outside the needle hub 103, the second support member 302 is located outside the actuating assembly 304, a straight line M in an axial direction of the first support member 301 and a straight line N in an axial direction of the second support member 302 are oppositely arranged in the direction perpendicular to the axial direction, the first support member and the second support member are used for supporting the actuating assembly 304 and the needle hub 103 in the direction perpendicular to the axial direction, and the actuating assembly 304 and the needle hub 103 that are connected to each other can axially move between the first support member 301 and the second support member 302. When moving towards the proximal end, the first support member 301 drives the needle hub 103 to move towards the proximal end, and the needle hub 103 drives the actuating assembly 304 to move towards the proximal end until the actuating assembly 304 is detached from the needle hub 103. After the two components are separated, the first support member 301 can still drive the needle hub 103 to continue to move towards the proximal end.

In this embodiment, the actuating assembly 304 may include a first gear 1021 and a first rack 1022 that mesh with each other. In this case, the first rack 1022 and the needle hub 103 are detachably connected in the direction perpendicular to the axial direction. After the first rack 1022 is connected to the needle hub 103 (as shown in Fig. 15a), the first support member 301 is located outside the needle hub 103 (that is, below the needle hub 103 as shown in the figure), the second support member 302 is located outside the first rack 1022 (that is, above the first rack 1022 as shown in the figure), the straight line M in the axial direction of the first support member 301 and the straight line N in the axial direction of the second support member 302 are oppositely arranged in the direction perpendicular to the axial direction, to support the first rack 1022 and the needle hub 103 in the direction perpendicular to the axial direction, so that the first gear 1022 and the needle hub 103 that are connected to each other can axially move between the first support member 301 and the second support member 302. When moving towards the proximal end, the first support member 301 drives the needle hub 103 to move towards the proximal end, and the needle hub 103 drives the first rack 1022 to move towards the proximal end until the first rack 1022 is detached from the needle hub 103. After the two components are separated, the first support member 301 can still drive the needle hub 103 to continue to move towards the proximal end.

In another embodiment, the actuating assembly 304 may not adopt the gear and the rack that mesh with each other, and the actuating assembly 304 may be a connecting structure. By pulling or pushing the connecting structure, an axial actuation function is achieved, thereby controlling the extension and retraction of the suture needle 3. The connecting structure may be a rod-shaped or a block-shaped structure.

Firstly, the first support member 301 and the second support member 302 are arranged in this embodiment. After the actuating assembly 304 is connected to the needle hub 103, the first support member 301 is located outside the needle hub 103, the second support member 302 is located outside the actuating assembly 304, the straight line M in the axial direction of the first support member 301 and the straight line N in the axial direction of the second support member 302 are oppositely arranged in the direction perpendicular to the axial direction, and the first support member and the second support member are used for supporting the actuating assembly 304 and the needle hub 103 in the direction perpendicular to the axial direction. To be specific, the first support member 301 is used for supporting the needle hub 103, the second support member 302 is used for supporting the actuating assembly 304, and the first support member 301, the needle hub 103, the actuating assembly 304, and the second support member 302 support each other on the same plane perpendicular to the axial direction, thereby preventing the four components from being detached from each other in the direction perpendicular to the axial direction without affecting the axial movement of the actuating assembly 304 and the needle hub 103 that are connected to each other between the first support member 301 and the second support member 302. Consequently, this does not affect the axially moving actuating assembly in controlling the extension and retraction of the suture needle 3.

Secondly, the first support member 301 is movably connected to the needle hub 103, and the actuating assembly 304 and the needle hub 103 are detachably connected in the direction perpendicular to the axial direction. When moving towards the proximal end, the first support member 301 drives the needle hub 103 to move towards the proximal end. In this case, the first support member 301, the needle hub 103, the actuating assembly 304, and the second support member 302 still support each other on the same plane perpendicular to the axial direction. Even if axial displacement of the four components occurs, the four components are still not detached from each other in the direction perpendicular to the axial direction. In addition, the needle hub 103 can drive the actuating assembly 304 to move towards the proximal end. Because the actuating assembly 304 controls the extension and retraction of the suture needle 3 through the needle hub 103, before the actuating assembly 304 is detached from the needle hub 103, the needle hub 103 can drive the actuating assembly 304 to move towards the proximal end first, thereby reducing interference from the actuating assembly 304 with other components of the suture apparatus 100 and enhancing operational performance.

Thirdly, by connecting the needle hub 103 to the suture needle 3, the actuating assembly 304 can be detached from the needle hub 103, so that the suture needle 3 with the suture thread 5 already pulled out (namely, the suture needle 3 with the suture thread 5 that has been already used for suturing in vivo) can be extracted from the body. In addition, by extracting the suture needle 3 from the body, a portion connected to the suture needle 3 of the suture thread 5 can be withdrawn from the body. This makes it convenient for the operator to deliver a knotting device into the body along the portion of the suture thread 5 that has been withdrawn from the body, or to directly knot the portion of the suture thread 5 that has been withdrawn from the body and push it into the body to complete the suturing and fixation.

When applied to a specific embodiment, the "the actuating assembly 304 can be driven first to move towards the proximal end, thereby reducing interference from the actuating assembly 304 with other components of the suture apparatus 100" mentioned above includes, but is not limited to, the following cases: referring to Fig. 12b, when the first control member 9 is in the locked state, the first rack 1022 of the second control assembly 102 enters the through hole 93 and axially moves until the second control assembly 102 controls the suture needle 3 to complete suturing. In this case, the first rack 1022 needs to be extracted from the through hole 93, that is, the first rack 1022 needs to be driven to move towards the proximal end. In this way, the first control member 9 can restore to the initial state, thereby restoring the suture arm 4 to the delivered state and conveniently withdrawing the suture arm from the body. Therefore, in the process of withdrawing the suture needle, the actuating assembly 304 can be driven to move towards the proximal end, which can reduce interference from the actuating assembly 304 with other components of the suture apparatus 100 and has practical significance.

Referring to Fig. 16a, Fig. 16b, and Fig. 16c, on one embodiment, the suture apparatus 100 further includes a sleeve-shaped structure 401. The sleeve-shaped structure 401 includes a first support member 301. The sleeve-shaped structure 401 is movably connected to the needle hub 103, a proximal end of the needle hub 103 is arranged inside the sleeve-shaped structure 401, and a distal end of the needle hub 103 can extend out of or can be retracted into the sleeve-shaped structure 401. The first support member 301 may be a side wall of the sleeve-shaped structure 401, or may be an element arranged on a side wall of the sleeve-shaped structure 401.

In one embodiment, one of the sleeve-shaped structure 401 and the needle hub 103 is provided with a through hole structure 1030, the other one of the sleeve-shaped structure 401 and the needle hub 103 is provided with a fixing structure 1033. The fixing structure 1033 is restricted to move inside the through hole structure 1030, to achieve the movable connection between the sleeve-shaped structure 401 and the needle hub 103, namely, the movable connection between the first support member 301 and the needle hub 103. In an embodiment, the sleeve-shaped structure 401 is provided with a long strip-shaped through hole structure 1030, and the needle hub 103 is provided with the fixing structure 1033. The fixing structure 1033 is a hook-shaped structure 10331, and the hook-shaped structure 10331 can abut against an inner wall 10301 of the through hole structure 1030, so that the hook-shaped structure 10331 is restricted to axially move inside the long strip-shaped through hole structure 1030.

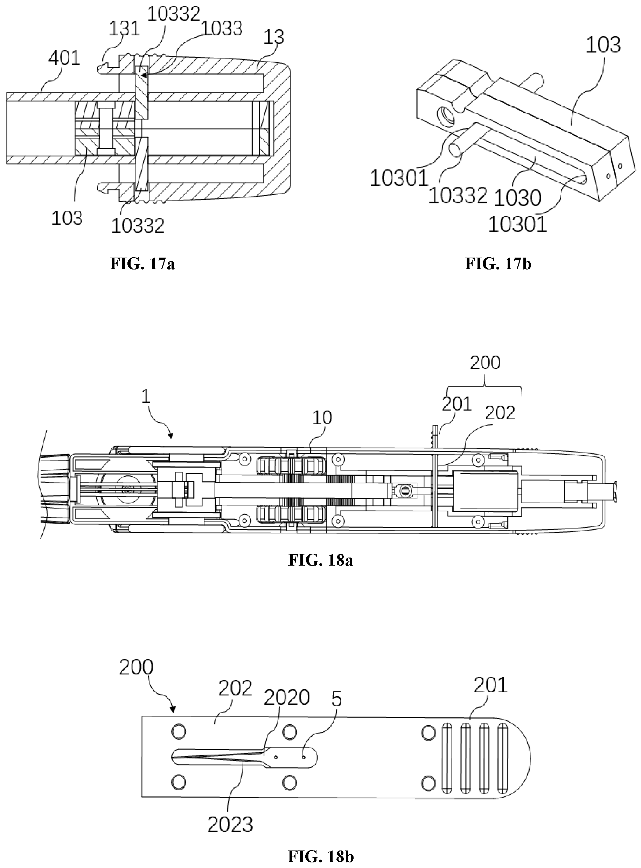

Referring to Fig. 17a and Fig. 17b, in another embodiment, the sleeve-shaped structure 401 is provided with the fixing structure 1033, the fixing structure 1033 is a rod-shaped structure 10332, and the needle hub 103 is provided with the long strip-shaped through hole structure 1030. The rod-shaped structure 10332 is inserted into the long strip-shaped through hole structure 1030 and can abut against the inner wall 10301 of the through hole structure 1030, so that the rod-shaped structure 10332 is restricted to axially move inside the long strip-shaped through hole structure 1030. If multiple needle hubs 103 are arranged, multiple fixing structures 1033 may be arranged and separately restricted inside different through hole structures 1030, to achieve separate movement of the multiple needle hub 103.

In another embodiment, the first support member 301 might not be arranged on the sleeve-shaped structure 401, and the first support member 301 may be a planar structure such as a flat plate, rod, strip, or block in any shape. A connecting member is arranged on the planar structure, to achieve the movable connection between the planar structure and the needle hub 103.

Referring to Fig. 3, the handle includes a handle housing 10 and a sleeve-shaped structure housing 13 that are detachably connected. Referring to Fig. 16 and Fig. 17a, the sleeve-shaped structure housing 13 is connected to the sleeve-shaped structure 401, and the sleeve-shaped structure housing 13 is arranged outside the sleeve-shaped structure 401. A connecting member 131 is arranged on the sleeve-shaped structure housing 13, and the connecting member 131 is inserted into the handle housing 10, so that the handle housing 10 is fixedly connected to the sleeve-shaped structure housing 13. A slit 132 is formed in the sleeve-shaped structure housing 13 at a position where the connecting member 131 is arranged, and the slit 132 allows movement of the position where the connecting member 131 is arranged in the sleeve-shaped structure housing 13 relative to other positions in the sleeve-shaped structure housing 13. When the handle housing 10 needs to be fixed to the sleeve-shaped structure housing 13, the position where the connecting member 131 is arranged in the sleeve-shaped structure housing 13 is pressed inward in the direction perpendicular to the axial direction, so that the connecting member 131 is easily inserted into the handle housing 10. After being inserted into the handle housing 10, the connecting member 131 can restore outward to an original shape in the direction perpendicular to the axial direction, so that the connecting member 131 can be engaged with the handle housing 10 in the direction perpendicular to the axial direction. In this way, the handle housing 10 is fixed to the sleeve-shaped structure housing 13. When the sleeve-shaped structure housing 13 needs to be detached from the handle housing 10, the position where the connecting member 131 is arranged in the sleeve-shaped structure housing 13 is pressed inward in the direction perpendicular to the axial direction, so that the connecting member 131 is easily detached from the handle housing 10. Then, the sleeve-shaped structure housing 13 is pulled out, to achieve detachment.

Referring to Fig. 15a, Fig. 15b, and Fig. 15c, in one embodiment, the second support member 302 is arranged on an inner wall of the handle housing 10. The actuating assembly 304 is provided with a first groove 3041, and the second support member 302 can be accommodated in the first groove 3041. When moving towards the proximal end, the first support member 301 drives the needle hub 103 to move towards the proximal end, and the needle hub 103 drives the actuating assembly 304 to move towards the proximal end until the second support member 302 is accommodated in the first groove 3041, so that the actuating assembly 304 is detached from the needle hub 103. Specifically, as shown in Fig. 15a, in this case, the sleeve-shaped structure housing 13 is not detached from the handle housing 10 yet, the second support member 302 is not accommodated in the first groove 3041, the first support member 301, the needle hub 103, the actuating assembly 304, and the second support member 302 support each other on the same plane perpendicular to the axial direction, and the actuating assembly 304 and the needle hub 103 that are connected to each other can axially move between the first support member 301 and the second support member 302. As shown in Fig. 15b, in this case, the sleeve-shaped structure housing 13 is pulled towards the proximal end, the sleeve-shaped structure housing 13 is detached from the handle housing 10, the second support member 302 is not accommodated in the first groove 3041, and the first support member 301, the needle hub 103, the actuating assembly 304, and the second support member 302 still support each other on the same plane perpendicular to the axial direction. The actuating assembly 304 has been already removed from the sleeve-shaped structure housing 401 completely at this moment, and the needle hub 103 is still stationary relative to the housing 13. As shown in Fig. 15c, as the sleeve-shaped structure housing 13 is further pulled towards the proximal end, the sleeve-shaped structure 401 drives the needle hub 103 to move towards the proximal end, the needle hub 103 drives the actuating assembly 304 to move towards the proximal end until the second support member 302 is accommodated in the first groove 3041, and the force balance among the first support member 301, the needle hub 103, the actuating assembly 304, and the second support member 302 on the same plane perpendicular to the axial direction is gradually disrupted, especially the force applied by the second support member 302 to the portion connected to the needle hub 103 of the actuating assembly 304 is gradually reduced. Because the magnitude of the force applied by the needle hub 103 to the actuating assembly 304 in the direction perpendicular to the axial direction and towards the actuating assembly 304 does not change significantly, the actuating assembly 304 tilts at a position in contact with the needle hub 103 of the actuating assembly 304 in the direction perpendicular to the axial direction (namely, a direction d) with the second support member 302 as the fulcrum. Because the actuating assembly 304 has been already completely removed from the sleeve-shaped structure 401, the actuating assembly 304 can be smoothly detached from the needle hub 103.

Further, one of the actuating assembly 304 and the needle hub 103 is provided with a raised portion 22, and the other one of the actuating assembly 304 and the needle hub 103 is provided with a recessed portion 33. The raised portion 22 and the recessed portion 33 are matched with each other, to allow the detachable connection between the actuating assembly 304 and the needle hub 103 in the direction perpendicular to the axial direction. This can ensure an effective connection when one of the actuating assembly 304 and the needle hub 103 drives the other one to move. In another embodiment, the raised portion 22 and the recessed portion 33 may be omitted, and the connection between the actuating assembly 304 and the needle hub 103 can only be ensured through the friction between the actuating assembly 304 and the needle hub 103. It may be understood that a contact surface of the actuating assembly 304 or/and the needle hub 103 may be arranged as a rough surface.

In another embodiment, the sleeve-shaped structure 401 further includes a second support member 302, that is, the second support member 302 is also arranged on the sleeve-shaped structure 401. In this case, a contact position between the needle hub 103 and the actuating assembly 304 needs to be located inside the sleeve-shaped structure 401. After the sleeve-shaped structure 401 is detached, the needle hub 103 can be detached from the actuating assembly 304. Specifically, the sleeve-shaped structure 401 is pulled towards the proximal end, to drive the needle hub 103 to move towards the proximal end, so that the actuating assembly 304 can also move towards the proximal end until the actuating assembly 304 cannot continue to move towards the proximal end. In this case, a locking member 3040 is arranged to abut against the actuating assembly 304, for example, the locking member 3040 inside the handle housing 10 abuts against the raised portion 10220 of the first rack 1022 in the axial direction, to prevent the actuating assembly 304 from moving too far towards the proximal end. The actuating assembly 304 is detached from the sleeve-shape structure 401, so that the needle hub 103 is detached from the actuating component 304. In this embodiment, the foregoing raised portion 22 and the foregoing recessed portion 33 may be arranged. In this case, the raised portion 22 and the recessed portion 33 may be elastic structures. The sleeve-shaped structure housing 13 is pulled towards the proximal end, the sleeve-shaped structure 401 drives the needle hub 103 to move towards the proximal end, and the needle hub 103 drives the actuating assembly 304 to move towards the proximal end. The sleeve-shaped structure housing 13 is further pulled towards the proximal end until the connection position between the needle hub 103 and the actuating assembly 304 is detached from the sleeve-shaped structure 401. Because the raised portion 22 and the recessed portion 33 are arranged, the connection still exists between the needle hub 103 and the actuating assembly 304 at this moment. Because the raised portion 22 or the recessed portion 33 is the elastic structure, the sleeve-shaped structure housing 13 is further pulled towards the proximal end, and during axial tension between the raised portion 22 and/or the recessed portion 33, the raised portion 22 can be easily detached from the recessed portion 33, to achieve detachment of the needle hub 103 from the actuating assembly 304. In another embodiment, the raised portion 22 and the recessed portion 33 may be omitted, and the connection between the actuating assembly 304 and the needle hub 103 may only be ensured through the friction between the actuating assembly 304 and the needle hub 103. It may be understood that a contact surface of the actuating assembly 304 or/and the needle hub 103 may be arranged as a rough surface.

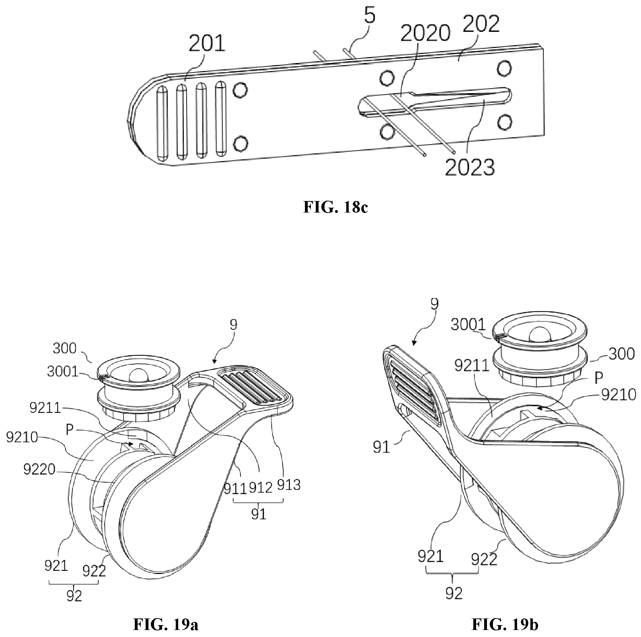

Referring to Fig. 18a, Fig. 18b, and Fig. 18c, in one embodiment, the suture apparatus 100 further includes a thread cutter 200. The thread cutter 200 is arranged on the suture apparatus 100, and the thread cutter 200 includes a holding portion 201 and a cutting portion 202 that are connected to each other. The suture thread 5 extends through the cutting portion 202, the holding portion 201 is pulled to fit the suture thread 5 against the cutting portion 202, so as to cut the suture thread 5. In this embodiment, the thread cutter 200 is arranged inside the handle 1, the holding portion 201 is arranged inside the handle housing 10, and the cutting portion 202 is arranged outside the handle housing 10. When the suture needle 3 is extracted together with the suture thread 5, the suture thread 5 withdrawn outside the body is cut short to avoid the excessively long suture thread 5. The cutting portion 202 is provided with a strip-shaped hole 2020, and a blade 2023 is arranged on an inner wall of the strip-shaped hole 2020.

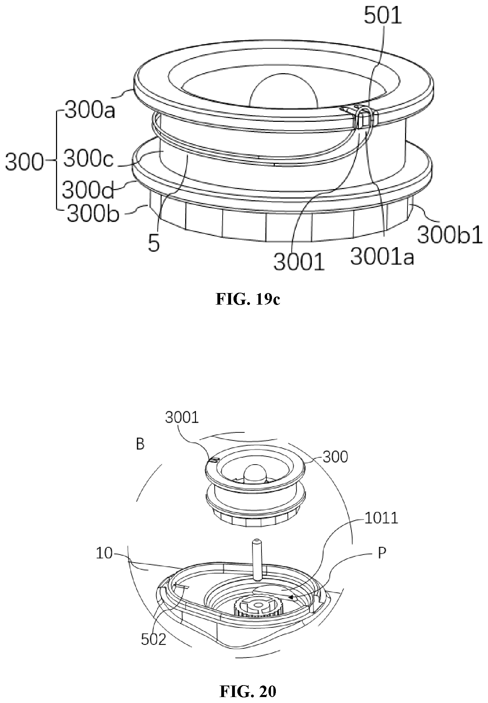

Referring to Fig. 3, Fig. 19a, Fig. 19b, and Fig. 19c, in one embodiment, the suture apparatus 100 further includes a thread spool 300. The suture thread 5 is wound around the thread spool 300, the thread spool 300 is rotated to release the suture thread 5, and the first control member 9 controls tightening or loosening of the thread spool 300. The thread spool 300 has a tightened state and a loosened state, and the first control member 9 has an initial state and a locked state.

Referring to Fig. 19a, when the first control member 9 is in the initial state, the thread spool 300 is in the tightened state. Referring to Fig. 19b, when the first control member 9 is in the locked state, the thread spool 300 is in the loosened state. The suture thread 5 is wound around the thread spool 300. One portion of the suture thread 5 is wound around the thread spool 300, another portion of the suture thread 5 extends to the suture arm 4 along the sheath 2, so that at least one end of the suture thread 5 is connected to the suture arm 4. In one embodiment, the suture thread 5 is folded in half, two ends of the suture thread are connected to the suture arm 4, and a folded position of the suture thread 5 is located on the thread spool 300. In another embodiment, one end of the suture thread 5 is connected to the suture arm 4, and the other end of the suture thread 5 is located on the thread spool 300.

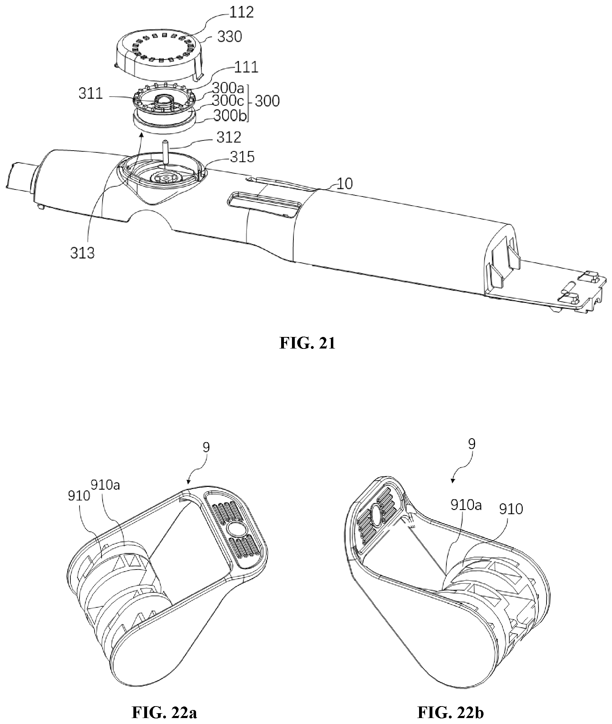

Refer to Fig. 19c again, in one embodiment, the thread spool 300 includes an upper convex ring 300a, a lower convex ring 300b, a thread winding portion 300c, and a separator 300d. The thread winding portion 300c is located between the upper convex ring 300a and the lower convex ring 300b, and the upper convex ring 300a and the lower convex ring 300b are used for limiting the position where the suture thread 5 is wound. The lower convex ring 300b is further matched with the first control member 9 to achieve the tightening or loosening of the thread spool 300 described in the foregoing embodiments. A damping structure is further arranged on the lower convex ring 300b. For example, an outer surface of the lower convex ring 300b is arranged as a polyhedral surface 300b1, to increase the tightness of matching between the lower convex ring 300b and the first control member 9. In addition, the separator 300d may be used for separating the thread wounding portion 300c from the lower convex ring 300b, to prevent the matching between the lower convex ring 300b and the first control member 9 from affecting the release of the suture thread 5 from the thread winding portion 300c. Referring to Fig. 21, in another embodiment, the separator 300d may be omitted.

Referring to Fig. 19c, a thread hooking member 3001 is arranged on the thread spool 300. The thread hooking member 3001 may be arranged at one or more positions on the upper convex ring 300a, the lower convex ring 300b, the thread winding portion 300c, and the separator 300d. After the suture thread 5 is folded in half, two ends of the suture thread 5 are connected to the suture arm 4, and the folded position 501 of the suture thread 5 is located on the thread spool 300. In this case, to facilitate winding the folded suture thread 5 around the thread spool 300, the folded position 501 of the suture thread 5 is first hooked onto the thread hooking member 3001, and then the folded suture thread 5 is wound around the thread spool 300 with the folded position 501 of the suture thread 5 that is hooked onto the thread hooking member 3001 as a starting point, to complete the winding of the suture thread 5. Then, another portion of the suture thread 5 extends through a threading hole 502 (as shown in Fig. 20) in the handle housing 10, and extends to the suture arm 4 along the sheath 20 and is connected to the suture arm 4. In this embodiment, the thread hooking member 3001 is a small bump 3001a. Specifically, the small bump 3001a is formed by cutting the upper convex ring 300a, and the small bump 3001a is separated from other portions of the upper convex ring 300a. In another embodiment, the thread hooking member 3001 may be a hook-shaped structure, a rod-shaped structure, or the like on the thread spool 300 or additionally installed on the thread spool 300, as long as the thread hooking member can hook the folded position 501 of the suture thread 5.

In this embodiment, the first control member 9 may be further used for controlling the suture arm 4 to switch between the delivered state and the expanded state. For a specific control process, refer to the foregoing embodiments. When the first control member 9 is in the initial state, the suture arm 4 is in the delivered state, and the thread spool 300 is in the tightened state. When the first control member 9 is in the locked state, the suture arm 4 is in the expanded state, and the thread spool 300 is in the loosened state. In another embodiment, the first control member 9 may be only used for controlling the tightening and loosening of the thread spool 300, and the suture arm 4 may be controlled by another element. Alternatively, the suture arm 4 is covered with a protective structure, the suture arm 4 is always in the expanded state, the state of the suture arm 4 does not need to be controlled, and the protective structure is removed after the suture arm reaches a target position.

In this embodiment, the first control member 9 having the initial state and the locked state is arranged. The first control member 9 controls the thread spool 300 to switch between the tightened state and the loosened state, to prevent the thread spool 300 from being always in the loosened state and releasing an excessively long suture thread 5. The excessively long suture thread 5 easily accumulates and becomes knotted, and easily interferes with other operations of the suture apparatus 100. The thread spool 300 is loosened as needed, so that operability and controllability are high.

Referring to Fig. 19a and Fig. 19b, in one embodiment, the first control member 9 includes a main body portion 92. The main body portion 92 includes a first main body member 921 and a second main body member 922, and the first main body member 921 and the second main body member 922 are oppositely arranged. The thread spool 300 is arranged between the first main body member 921 and the second main body member 922. One side close to the thread spool 300 of the first main body member 921 has a first main body member inner wall 9210, one side close to the thread spool 300 of the second main body member 922 has a second main body member inner wall 9220, and the position where the thread spool 300 is arranged between the first main body member 921 and the second main body member 922 is a first position P. At the first position P, the distance between the first main body member inner wall 9210 and the second main body member inner wall 9220 when the first control member 9 is in the initial state is shorter than the distance between the first main body member inner wall 9210 and the second main body member inner wall 9220 when the first control member 9 is in the locked state, so that the tightening and loosening of the thread spool 300 is achieved in a radial direction of the thread spool 300. When the distance between the first main body member inner wall 9210 and the second main body member inner wall 9220 is short, the first main body member inner wall and the second main body member inner wall are used for clamping the thread spool 300, to keep the thread spool 300 in the tightened state. When the distance between the first main body member inner wall 9210 and the second main body inner wall 9220 is long, the first main body member inner wall and the second main body member inner wall are used for loosening the thread spool 300, to keep the thread spool 300 in the loosened state.

In this embodiment, a recess 9211 is arranged at a partial position of the first main body member inner wall 9210, to achieve a long distance between the first main body member inner wall 9210 and the second main body member inner wall 9220. When the first control member 9 is in the initial state, at the first position P, a portion of the first main body member inner wall 9210 that is not provided with the recess 9211 abuts against the thread spool 300. When the first control member 9 is in the locked state, at the first position P, a portion of the first main body member inner wall 9210 that is provided with the recess 9211 abuts against the thread spool 300. In another embodiment, the second main body member inner wall 9220 may be also provided with a recess, or only the second main body member inner wall 9220 is provided with a recess. In another embodiment, the overall thickness of the first main body member 921/the second main body member 922 is uniform, and the recess is omitted, as long as at the position P, the distance between the first main body member 921 and the second main body member 922 is short when the first control member 9 is in the initial state, and the distance between the first main body member 921 and the second main body member 922 is long when the first control member 9 is in the locked state.

In this embodiment, the first operating portion 91 is operated to drive the first main body member 921 and the second main body member 922 that are connected to each other to rotate. In another embodiment, different operating portions are arranged, one of the first main body member 921 and the second main body member 922 can rotate, and the thread spool 300 is tightened and loosened by changing the distance between the two main body member inner walls. In another embodiment, the operating portion may be omitted, and the first main body member 921 or/and the second main body member 922 is directly rotated.

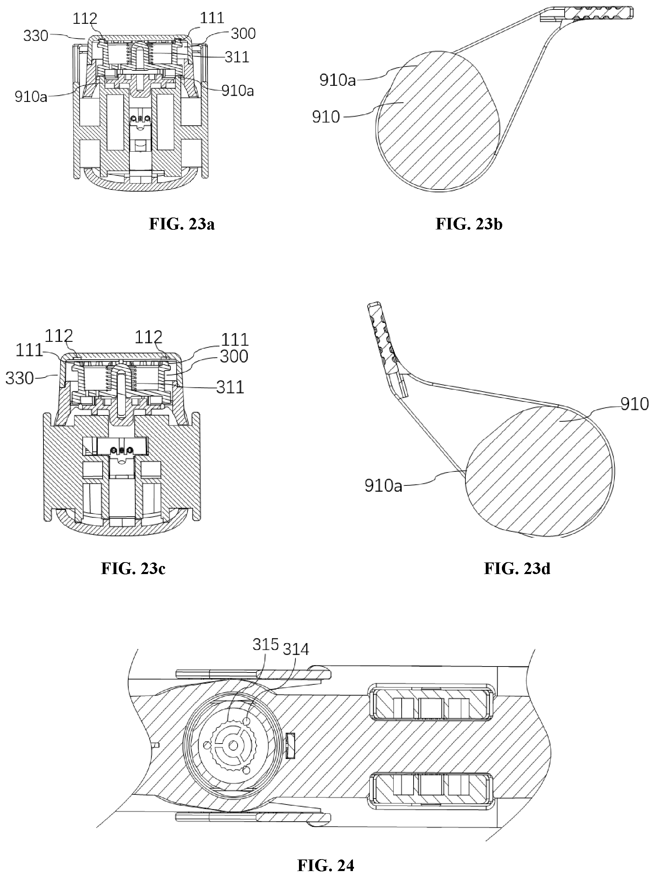

Referring to Fig. 3, Fig. 19, and Fig. 20, in one embodiment, the thread spool 300 may be arranged on an exterior of the handle housing 10, and the main body portion 92 is arranged on an interior of the handle housing 10. At the first position P, the handle housing 10 is provided with an open hole 1011, and the first main body member inner wall 9210 can abut against the thread spool 300 through the open hole 1011. It may be understood that at the first position P, the handle housing 10 may be further provided with another open hole 1011, and the second main body member inner wall 9220 can abut against the thread spool 300 through the open hole 1011. Referring to Fig. 21, Fig. 22, Fig. 23, in one embodiment, the suture apparatus 100 further includes a fixed cover portion 330. The fixed cover portion 330 covers an exterior of the thread spool 300. Referring to Fig. 23a and Fig. 23b, when the first control member 9 is in the initial state, the first control member 9 presses against one end of the thread spool 300, and the other end of the thread spool 300 is fixed to the fixed cover portion 330. Referring to Fig. 23c and Fig. 23d, when the first control member 9 is in the locked state, the other end of the thread spool 300 is detached from the fixed cover portion 330. Through such an arrangement, the tightening and loosening of the thread spool 300 can be achieved in a height direction of the thread spool 300. In this embodiment, the fixed cover portion 330 is fixedly connected to the handle housing 10, so that the fixed cover portion 330 remains fixed.

In this embodiment, the first control member 9 includes an ejector 910. An ejecting portion 910a is arranged at an end of the ejector 910, and the first control member 9 is rotatable. When the first control member 9 is in the initial state, the ejecting portion 910a approaches an end of the thread spool 300 and presses against the thread spool 300, to tighten the thread spool 300. When the first control piece 9 is rotated to the locked state, the ejecting portion 910a leaves away from the end of the thread spool 300, to loosen the thread spool 300.

Further, the suture apparatus 100 includes an elastic member 311. Two ends of the elastic member 311 are respectively connected to the fixed cover portion 330 and the thread spool 300. When the first control member 9 is in the initial state, the ejecting portion 910a approaches the end of the thread spool 300 and presses against the thread spool 300 to tighten the thread spool 300. In this case, the elastic member 311 is compressed, thereby storing elastic potential energy. When the first control member 9 is rotated to the locked state, the ejecting portion 910a leaves away from the end of the thread spool 300. The compressed elastic member 311 releases the elastic potential energy. The elastic potential energy acts on the thread spool 300, thereby accelerating the detachment of the thread spool 300 from the fixed cover portion 330 and accelerating the loosening of the thread spool 300.

Further, a first fixing portion 111 is arranged at the other end of the thread spool 300, and the fixed cover portion 330 is provided with a second fixing portion 112. One of the first fixing portion 111 and the second fixing portion 112 is a groove, and the other one of the two components is a bump. The bump is connected to the groove, thereby improving capability of the fixed cover portion 330 to tighten the thread spool 300.

In one embodiment, by the foregoing methods, the tightening and loosening of the thread spool 300 can be achieved in both the radial direction of the thread spool 300 and the height direction of the thread spool 300. Specifically, the method includes, but is not limited to, arranging the ejector 910 on the main body portion 92. In another embodiment, the tightening and loosening of the thread spool 300 may be achieved in either the radial direction of the thread spool 300 or the height direction of the thread spool 300.

Referring again to Fig. 21, at least a portion of the thread spool 300 is arranged outside the handle housing 10, a first post 312 is arranged on the handle housing 10, the thread spool 300 is inserted into the first post 312, and the thread spool 300 rotates around the first post 312, to release the suture thread 5.

Referring to Fig. 21 and Fig. 24, an opening 313 is formed at an end close to the handle housing 10 of the thread spool 300, and a first damping structure 314 is arranged on an inner wall of the thread spool 300. That is, the first damping structure 314 is arranged on the inner wall surrounding the opening 313. The handle housing 10 is provided with a second damping structure 315 matched with the first damping structure 314. As the thread spool 300 rotates, the first damping structure 314 moves relative to the second damping structure 315, to reduce the rotation speed of the thread spool 300. In another embodiment, the first damping structure 314 is arranged at the end close to the handle housing 10 of the thread spool 300, and the handle housing 10 is provided with the second damping structure 315 matched with the first damping structure 314.

During operation, the distal end of the suture needle 3 is connected to the suture arm 4, to extract the at least one end of the suture thread 5 from the suture arm 4, then the suture needle 3 is displaced to drive displacement of the suture thread 5, and the suture thread 5 drives the thread spool 300 to rotate, so as to release the suture thread 5 wound around the thread spool 300. Because of the inertia of rotation of the thread spool 300, the thread spool 300 easily rotates too fast, which results in excessive release of the suture thread 5 from the thread spool. The excessively long suture thread easily accumulates and becomes knotted, and easily interferes with other operations of the suture apparatus 100. Therefore, the rotation speed of the thread spool 300 needs to be reduced, so that the suture thread 5 with a reasonable length can be released.

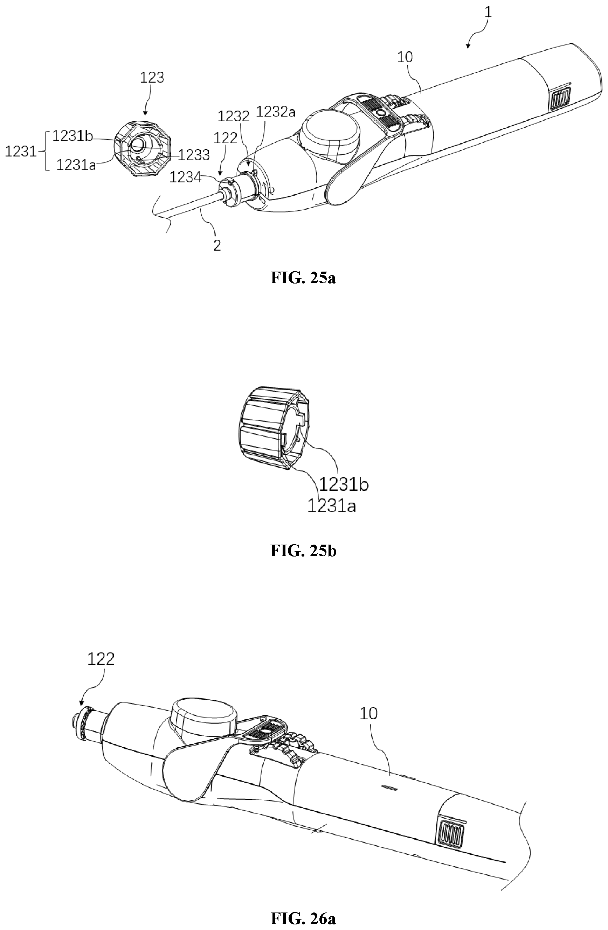

Referring to Fig. 25a, Fig. 26, and Fig. 27, in one embodiment, the handle 1 further includes a receiving structure 122. The receiving structure 122 is fixedly connected to the sheath 2, and the receiving structure 122 includes a first portion 1221. The first portion 1221 abuts against the handle housing 10, and the first portion 1221 is provided with a first matching structure 1222. The handle housing 10 is provided with a second matching structure 1223. One of the first matching structure 1222 and the second matching structure 1223 is at least one bump structure 1221a, and the other one of the first matching structure 1222 and the second matching structure 1223 is a plurality of recess structures 1221b. The plurality of recess structures 1221b are arranged in the circumferential direction. When rotated, the receiving structure 122 circumferentially moves relative to the handle housing 10, to cause one bump structure 1221a to sequentially move inside the plurality of recess structures 1221b.

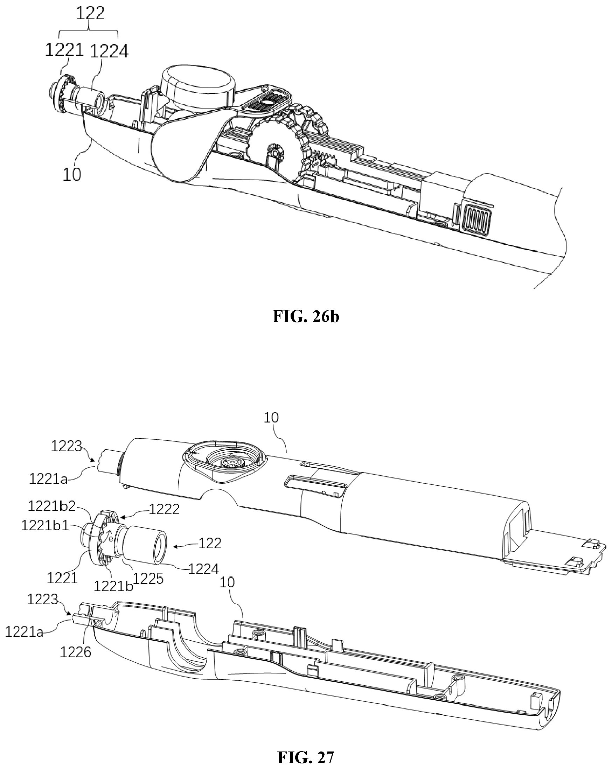

Referring to Fig. 27, in this embodiment, the receiving structure 122 is arranged. The receiving structure 122 is connected to the sheath 2, and when rotated, the receiving structure 122 moves circumferentially relative to the handle housing 10. In this way, after the sheath is delivered into the body, by adjusting a circumferential relative positional relationship between the receiving structure 122 and the handle housing 10, the sheath 2 is rotated and a circumferential position of the sheath 2 is adjusted, so that the sheath can be adapted to an in vivo environment more flexibly. In addition, other portions of the handle 1 can remain stationary, so that it is convenient to operate other portions of the handle 1. In addition, the first matching structure 1222 and the second matching structure 1223 are respectively arranged on the first portion 1221 of the receiving structure 122 and the handle housing 10, one of the first matching structure 1222 and the second matching structure 1223 is at least one bump structure 1221a, and the other one of the first matching structure 1222 and the second matching structure 1223 is the plurality of recess structures 1221b. The plurality of recess structures 1221b are arranged in the circumferential direction. When rotated, the receiving structure 122 circumferentially moves relative to the handle housing 10, to cause one bump structure 1221a to sequentially move inside the plurality of recess structure 1221b. In this way, during rotation, quantitative control of a circumferential rotation angle of the receiving structure 122 can be achieved, to precisely control a circumferential rotation angle of the sheath 2 and to improve the precision and success rate of operation. In this embodiment, the distal end of the sheath 2 has a bent portion, or does not have a bent portion. In Fig. 27, the first matching structure 1222 is the recess structure 1221b, the second matching structure 1223 is the bump structure 1221a. Referring to Fig. 28, in another embodiment, the first matching structure 1222 is the bump structure 1221a, and it may be understood that the second matching structure 1223 is the recess structure 1221b.

In one embodiment, the recess structure 1221b includes a first recess 1221b1 and a second recess 1221b2 that are connected to each other. The bump structure 1221a moves from the first recess 1221b1 to the second recess 1221b2 or moves from the second recess 1221b2 to the first recess 1221b1, to drive the sheath 2 to rotate clockwise or counterclockwise relative to the handle housing 10 by a first angle e. In this embodiment, the sheath can be rotated clockwise or counterclockwise, so that the rotation angle of the sheath 2 can be precisely adjusted. During operation, if the sheath is rotated by a large angle in a direction, the sheath can be reversibly adjusted or modified to a suitable angle.

In one embodiment, the receiving structure 122 further includes a second portion 1224. The second portion 1224 is fixedly connected to the sheath 2. The first portion 1221 is arranged on an outer surface of the second portion 1224, which facilitates the connection between the receiving structure 122 and the sheath 2 without affecting the matching between the receiving structure 122 and the handle housing 10. Specifically, at least a portion of the second portion 1224 is inserted into the handle housing 10, a proximal end of the first portion 1221 abuts against a distal end of the handle housing 10, the first matching structure 1222 is arranged at the proximal end of the first portion 1221, and the second matching structure 1223 is arranged at the distal end of the handle housing 10. In another embodiment, the receiving structure 122 may not include the second portion 1224 and only include the first portion 1221. One end of the first portion 1221 may be connected to the sheath 2, and the other end of the first portion 1221 is matched with the handle housing 10.

Referring to Fig. 25a and Fig. 25b, in one embodiment, the handle 1 further includes a knob 123. The knob 123 surrounds and is fixed on the receiving structure 122, so that the receiving structure 122 is conveniently rotated through the knob 123. In another embodiment, the knob 123 may be omitted, and the receiving structure 122 is directly rotated.

The knob 123 is provided with a knob stopping member 1231, and the handle housing 10 is provided with a housing stopping member 1232. The knob 123 surrounds the receiving structure 122, the knob 123 is rotated in a first direction Q, and the knob stopping member 1231 and the housing stopping member 1232 stop each other in the circumferential direction, to prevent the knob 123 from continuing to rotate in the first direction Q. The first direction Q may be a clockwise or counterclockwise direction.

The knob stopping member 1231 includes a first knob stopping member 1231a and a second knob stopping member 1231b, and the housing stopping member 1232 moves between the first knob stopping member 1231a and the second knob stopping member 1231b. Alternatively, the housing stopping member 1232 includes a first housing stopping member 1232a and a second housing stopping member 1232b, and the knob stopping member 1231 moves between the first housing stopping member 1232a and the second housing stopping member 1232b. Through such an arrangement, circumferential rotation between the knob stopping member 1231 and the housing stopping member 1232 can be controlled within a certain range of the entire circumference, so that the knob stopping member 1231 cannot be rotated a full circle relative to the housing stopping member 1232. That is, the knob 123 is rotated within the certain range of the entire circumference relative to the handle housing 10, thereby controlling the circumferential rotation angle of the sheath 2.

In another embodiment, the knob 123 has an initial state, the knob 123 is provided with a first positioning member 1233, and the receiving structure 122 is provided with a second positioning member 1234. When the knob 123 and the receiving structure 122 are connected to each other, the first positioning member 1233 and the second positioning member 1234 are connected and fixed to each other, so that the knob 123 is in the initial state when matched with the handle housing 10. During installation, the receiving structure 122 may be first connected to the handle housing 10, and then the knob 123 is connected to the receiving structure 122 through matching between the first positioning member 1233 and the second positioning member 1234. Therefore, by controlling an initial connection position between the knob 123 and the receiving structure 122, the initial connection position between the knob stopping member and the handle housing 10 can be controlled. Further, the circumferential distance between the knob stopping member 1231 and the housing stopping member 1232 can be controlled, to control the rotation angle range of the knob 123, so as to precisely control a circumferential rotation angle range of the sheath 2. In another embodiment, the first knob stopping member 1231a, the second knob stopping member 1231b, the first positioning member 1233, and the second positioning member 1234 are all arranged. Alternatively, the first housing stopping member 1232a, the second housing stopping member 1232b, the first positioning member 1233, and the second positioning member 1234 are all arranged, so that the circumferential rotation angle range of the sheath 2 can be precisely controlled.

In another embodiment, it may be understood that a stopping member may be further arranged on the receiving structure 122. Specifically, it may be understood that the receiving structure 122 may be provided with a receiving structure stopping member, and the handle housing 10 is provided with the housing stopping member 1232. The knob 123 is rotated in the first direction, and the receiving structure stopping member and the housing stopping member 1232 stop each other in the circumferential direction, to prevent the receiving structure from continuing to rotate in the first direction.

Referring to Fig. 27, to better fix the receiving structure 122 to the handle housing 10, fixing elements 1225 and 1226 are respectively arranged on the receiving structure 122 and the handle housing 10. The fixing element 1225 of the receiving structure 122 is a groove structure, the fixing element 1226 of the handle housing 10 is a bump structure, and the groove structure and the bump structure are matched with and connected to each other. Referring to Fig. 28, in one embodiment, the fixing element 1225 of the receiving structure 122 is a bump structure, the fixing element 1226 of the handle housing 10 is a groove structure, and the groove structure and the bump structure are matched with and connected to each other.

In this embodiment where the receiving structure 122 is arranged, the receiving structure 122 may be applied to the suture apparatus 100. It may be understood that the receiving structure may alternatively applied to any delivery apparatus. Referring to Fig. 1, the delivery apparatus 1000 includes a handle 1 and a sheath 2 that are connected to each other.

An example in which the suture apparatus 100 of this embodiment is used for suturing a patent foramen ovale is used. There are two suture needles 3, which are a first suture needle 31 and a second suture needle 32, respectively. Referring to Fig. 29a, two ends 5a and 5b of the suture thread 5 are respectively located on two sides of the suture arm 4, and a middle portion 5c of the suture thread 5 extends towards the proximal end along the sheath 2 until the suture thread can be wound around the thread spool 300. A general operation process may be as follows: the suture apparatus 100 is delivered to a target position along a guide wire, the distal end of the suture apparatus 100 extends through the foramen ovale, and then the suture arm 4 is expanded. Two sides of the suture arm 4 respectively abut against the primary septum 600 and the secondary septum 700 at a position of the foramen ovale. Referring to Fig. 29b, the first suture needle 31 extends out to extend through the primary septum 600 and is connected to one side of the suture arm 4, so that one end 5a of the suture thread 5 is extracted from the suture arm 4. The second suture needle 32 extends out to extend through the secondary septum 700 and is connected to the other side of the suture arm 4, so that the other end 5b of the suture thread 5 is extracted from the suture arm 4. The first suture needle 31 and the second suture needle 32 respectively carry the two ends 5a and 5b of the suture thread 5 and slowly withdraw the two ends towards the proximal end to back to the sheath 2 until the two ends are withdrawn from the body. Meanwhile, the middle portion 5c of the suture thread 5 is slowly detached from the thread spool 300, and slowly moves towards the distal end until the middle portion reaches the position of the primary septum and the secondary septum (as shown in Fig. 29c). Finally, the knotting device is delivered into the body along the two ends 5a and 5b of the suture thread 5 to cut off any excess portion of suture thread 5 and complete the closure of the patent foramen ovale. To make the figures simpler and easier to understand, the first suture needle 31, the second suture needle 32, the suture arm 4, the sheath 2, the thread spool 300, and the like are not shown in Fig. 29a, Fig. 29b, and Fig. 29c. For the relevant structures, refer to the foregoing embodiments.

The above are merely preferred specific embodiments of the present invention, and the scope of protection of the present invention is not limited thereto. Changes or substitutions readily made by those skilled in the art within the technical scope disclosed in the present invention shall fall within the scope of protection of the present invention. Therefore, the scope of protection of the present invention is subject to the appended claims.

## Claims

1. A suture apparatus, comprising a suture needle, a suture arm, and a suture thread, wherein at least one end of the suture thread is located on the suture arm, a distal end of the suture needle is capable of being connected to the suture arm to extract the at least one end of the suture thread from the suture arm, the suture apparatus further comprises a actuating assembly, a needle hub, a first support member, and a second support member, and the first support member is movably connected to the needle hub;
the needle hub is connected to the suture needle, the actuating assembly and the needle hub are detachably connected in a direction perpendicular to an axial direction, the actuating assembly controls the extension and retraction of the suture needle through the needle hub, and when the suture needle extends out, the distal end of the suture needle is capable of being connected to the suture arm;
after the actuating assembly is connected to the needle hub, the first support member is located outside the needle hub, the second support member is located outside the actuating assembly, a straight line in an axial direction of the first support member and a straight line in an axial direction of the second support member are oppositely arranged in the direction perpendicular to the axial direction, the first support member and the second support member are used for supporting the actuating assembly and the needle hub in the direction perpendicular to the axial direction, and the actuating assembly and the needle hub that are connected to each other are capable of axially moving between the first support member and the second support member; and
when moving toward a proximal end, the first support member drives the needle hub to move towards the proximal end, and the needle hub drives the actuating assembly to move towards the proximal end until the actuating assembly is detached from the needle hub.

2. The suture apparatus according to claim 1, further comprising a sleeve-shaped structure, wherein the sleeve-shaped structure comprises the first support member, the sleeve-shaped structure is movably connected to the needle hub, a proximal end of the needle hub is arranged inside the sleeve-shaped structure, and a distal end of the needle hub is capable of extending out of or retracting into the sleeve-shaped structure.

3. The suture apparatus according to claim 2, wherein one of the sleeve-shaped structure and the needle hub is provided with a through hole structure, the other one of the sleeve-shaped structure and the needle hub is provided with a fixing structure, and the fixing structure is restricted to move inside the through hole structure.

4. The suture apparatus according to claim 2, further comprising a handle, wherein the handle comprises a handle housing and a sleeve-shaped structure housing that are detachably connected, the actuating assembly moves inside the handle housing, the sleeve-shaped structure housing is connected to the sleeve-shaped structure, and the sleeve-shaped structure housing is arranged outside the sleeve-shaped structure.

5. The suture apparatus according to claim 4, wherein a connecting member is arranged on the sleeve-shaped structure housing, the connecting member is inserted into the handle housing to fixedly connect the handle housing to the sleeve-shaped structure housing, a slit is formed in the sleeve-shaped structure housing at a position where the connecting member is arranged, and the slit allows movement of the position where the connecting member is arranged in the sleeve-shaped structure housing relative to other positions in the sleeve-shaped structure housing.

6. The suture apparatus according to claim 2, further comprising a handle, wherein the handle comprises a handle housing, and the second support member is arranged on an inner wall of the handle housing.

7. The suture apparatus according to claim 6, wherein the actuating assembly is provided with a first groove, the second support member is capable of being accommodated in the first groove, when moving towards the proximal end, the first support member drives the needle hub to move towards the proximal end, and the needle hub drives the actuating assembly to move towards the proximal end until the second support member is accommodated in the first groove, so as to detach the actuating assembly from the needle hub.

8. The suture apparatus according to claim 2, wherein the sleeve-shaped structure further comprises the second support member.

9. The suture apparatus according to claim 1, wherein one of the actuating assembly and the needle hub is provided with a raised portion, the other one of the actuating assembly and the needle hub is provided with a recessed portion, and the raised portion and the recessed portion are matched with each other to allow detachable connection between the actuating assembly and the needle hub in the direction perpendicular to the axial direction.

10. The suture apparatus according to claim 1, further comprising a thread cutter, the thread cutter is arranged on the suture apparatus, the thread cutter comprises a holding portion and a cutting portion connected to each other, the suture thread extends through the cutting portion, the holding portion is pulled to cause the suture thread to fit against the cutting portion, so as to cut the suture thread.

11. The suture apparatus according to claim 1, wherein the at least one end of the suture thread is located on the suture arm, the suture arm switches between a delivered state and an expanded state, and when the suture arm in the expanded state, the distal end of the suture needle is capable of being connected to the suture arm to extract the at least one end of the suture thread from the suture arm;
the suture apparatus further comprises a first connecting member, a second connecting member, a sliding block assembly, and a first control member, the sliding block assembly comprises a first sliding block, a second sliding block, and an elastic member that are connected to each other, the second sliding block and the elastic member are axially arranged inside the first sliding block, the second sliding block is capable of moving relative to the first sliding block, two ends of the first connecting member are respectively connected to the first sliding block and the first control member, and two ends of the second connecting member are respectively connected to the suture arm and the second sliding block; and
the first control member has an initial state and a locked state, during movement of the first control member from the initial state to the locked state, the first control member drives the first sliding block to axially move in a first direction through the first connecting member to form a first movement stroke, the first sliding block drives the second sliding block to axially move in the first direction through the elastic member to form a second movement stroke, and the first movement stroke is greater than the second movement stroke to allow elastic deformation of the elastic member inside the first sliding block and cause the suture arm to gradually switch from the delivered state to the expanded state; and when the first control member is in the locked state, the first control member remains the elastic deformation of the elastic member through the first connecting member and the first sliding block, to fix the second sliding block and lock the suture arm in the expanded state.

12. The suture apparatus according to claim 1, further comprising a handle and a sheath that are connected to each other, wherein the handle comprises a handle housing and further comprises a receiving structure, the receiving structure is fixedly connected to the sheath, the receiving structure comprises a first portion, the first portion abuts against the handle housing, the first portion is provided with a first matching structure, the handle housing is provided with a second matching structure, one of the first matching structure and the second matching structure is at least one bump structure, the other one of the first matching structure and the second matching structure is a plurality of recess structures, the plurality of recess structures are arranged in a circumferential direction, and when rotated, the receiving structure is capable of circumferentially moving relative to the handle housing, to cause one bump structure to sequentially move inside the plurality of recess structures.

13. The suture apparatus according to claim 1, further comprising a first control member and a thread spool, wherein one portion of the suture thread is wound around the thread spool, and another portion of the suture thread extends to the suture arm to allow the connection between the at least one end of the suture thread and the suture arm; and
the first control member is capable of controlling tightening and loosening of the thread spool, the thread spool has a tightened state and a loosened state, the first control member has an initial state and a locked state, when the first control member is in the initial state, the thread spool is in the tightened state, and when the first control member is in the locked state, the thread spool is in the loosened state, and the thread spool in the loosened state is capable of releasing the suture thread.
